# EUROPEAN PATENT SPECIFICATION

(11) **EP 3 414 342 B1**
(45) Date of publication and mention of the grant of the patent: **03.07.2019**
(21) Application number: 17700631.9
(22) Date of filing: 09.01.2017
(51) Int. Cl.: C12Q 1/6804, C12Q 1/6825

(54) **MAGNETIC BEADS-BASED ELECTROCHEMICAL BIOSENSOR**
ELEKTROCHEMISCHER BIOSENSOR BASIEREND AUF MAGNETISCHEN PERLEN
BIOCAPTEUR ÉLECTROCHIMIQUE MAGNÉTIQUE À BASE DE BILLES

(30) Priority: 09.02.2016 EP 16382053
(43) Date of publication of application: 19.12.2018
(73) Proprietor: Mirnax Biosens, S.L., 14014 Córdoba (ES)
(72) Inventor: PINGARRÓN, José Manuel, 28040 Madrid (ES); CAMPUZANO, Susana, 28040 Madrid (ES); RUIZ-VALDEPEÑAS-MONTIEL, Víctor, 28040 Madrid (ES); TORRENTE-RODRÍGUEZ, Rebeca Magnolia, 28040 Madrid (ES); MONTOYA, Juan José, 28020 Madrid (ES)
(74) Representative: Hoffmann Eitle
(86) International application number: PCT/EP2017/050348
(87) International publication number: WO 2017/137192

(56) References cited:
- CN-A- 104 878 075
- US-A1- 2011 294 111
- US-A1- 2014 228 227
- US-A1- 2015 184 223
- R.M. TORRENTE-RODRÍGUEZ ET AL: "Simultaneous detection of two breast cancer-related miRNAs in tumor tissues using p19-based disposable amperometric magnetobiosensing platforms", BIOSENSORS AND BIOELECTRONICS, vol. 66, 26 November 2014 (2014-11-26), pages 385-391, XP055273130, NL ISSN: 0956-5663, DOI: 10.1016/j.bios.2014.11.047
- TORRENTE-RODRÍGUEZ R M ET AL: "Electrochemical bioplatforms for the simultaneous determination of interleukin (IL)-8 mRNA and IL-8 protein oral cancer biomarkers in raw saliva", BIOSENSORS AND BIOELECTRONICS, vol. 77, 14 November 2015 (2015-11-14), pages 543-548, XP029311835, ISSN: 0956-5663, DOI: 10.1016/J.BIOS.2015.10.016

## Description

### FIELD OF THE INVENTION

The present invention relates to the medical field, in particular to the detection and/or quantification of miRNA, RNA or DNA molecules of interest in at least one isolated complex clinical sample of different types of specimens by using a magnetic beads (MBs)-based electrochemical sensor.

### BACKGROUND OF THE INVENTION

MicroRNAs are small regulatory RNAs that are currently emerging as new biomarkers for cancer and other diseases. In order for biomarkers to be useful in clinical settings, they should be accurately and reliably detected in clinical samples such as formalin fixed paraffin embedded (FFPE) sections and blood serum or plasma. These types of samples represent a challenge in terms of microRNA quantification.

The newly developed method disclosed herein enables accurate and reproducible quantification of microRNAs in clinical samples, preferably in scarce clinical samples, and solves the problem of providing for a methodology capable of detecting and/or quantifying miRNA, RNA or DNA molecules of interest in at least one isolated complex clinical sample of different types of specimens, reliably, rapidly (in approx. less than 75 min, preferably in about 30 minutes), reproducibly and with high specificity and sensitivity.

R.M. TORRENTE-RODRíGUEZ ET AL, BIOSENSORS AND BIOELECTRONICS, vol. 66, 26 November 2014, pages 385-391 relates to simultaneous detection of two breast cancer-related miRNAs in tumor tissues using p19-based disposable amperometric magnetobiosensing platforms.

### BRIEF DESCRIPTION OF THE INVENTION

The present invention is defined by the appended claims.

The present invention solves the problem of providing for a methodology capable of detecting and/or quantifying miRNA, RNA or DNA molecules of interest in at least one isolated complex clinical sample of different types of specimens, reliably, rapidly (in aprox. less than 75 min, preferably in about 30 minutes), reproducibly and with high specificity and sensitivity.

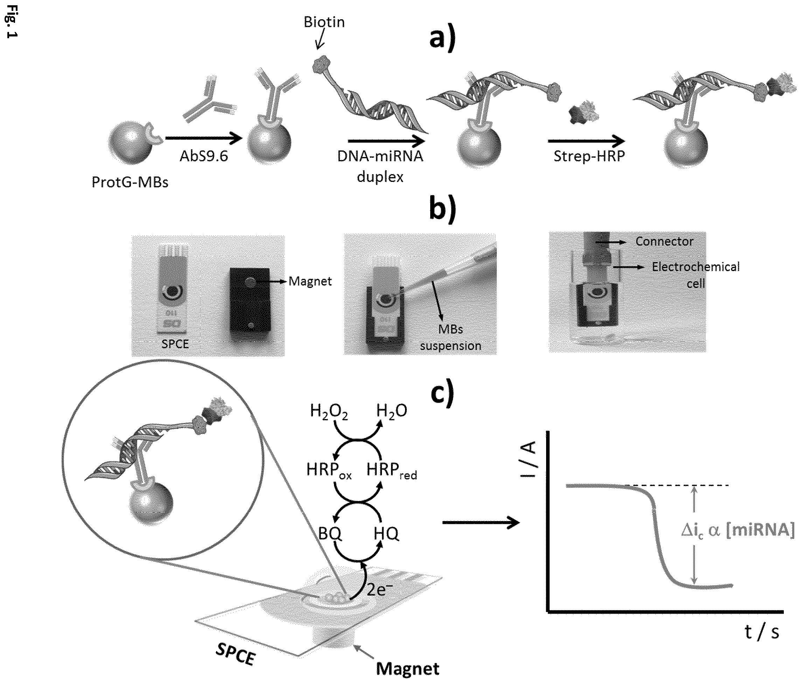

Basically the general procedure followed by the method of the present invention is illustrated in figure 1 and involves four main steps: (i) homogeneous hybridization of the synthetic DNA or RNA probe and the target miRNA, RNA or DNA molecule of interest; (ii) capture of the resultant DNA/RNA, DNA/DNA or RNA/RNA duplex by an antibody-modified magnetic particle, preferably MBs, in solution; (iii) enzymatic labeling of the biotinylated DNA/RNA, DNA/DNA or RNA/RNA duplex captured onto the antibody-modified magnetic particles, preferably MBs, wherein the antibodies or a fragment thereof are bound to the magnetic particles though a protein G coupled to the surface of the magnetic particles, and (iv) electrochemical detection of the modified-magnetic particles on an electrochemical sensor, preferably on screen-printed carbon electrodes (SPCEs). By using this procedure, up to 30 sensors a day can be worked (or more if the process is automated), illustrating the use of the present methodology for the rapid, simple, specific, quantitative and ultrasensitive detection and identification (detection limit [LOD] = 2.4 pM) of polynucleotides (endogenously single-stranded or previously denatured if naturally double-stranded) such as the miRNAs present in a given sample.

### BRIEF DESCRIPTION OF THE FIGURES

**FIG 1****.** Schematic representation of the AbS9.6-MBs-based amperometric biosensor designed for the determination of a target miRNA (miRNA-21 or miRNA-205). The components of the MBs-based biosensor are not drawn to scale (a). Picture showing the SPCE (screen-printed carbon electrode) and the homemade magnet holding block, and the modified MBs on the SPCE assembled on the magnet holding block and in the specific cable connector (b). Reactions involved in the electrochemical detection and amperometric signal obtained (c).

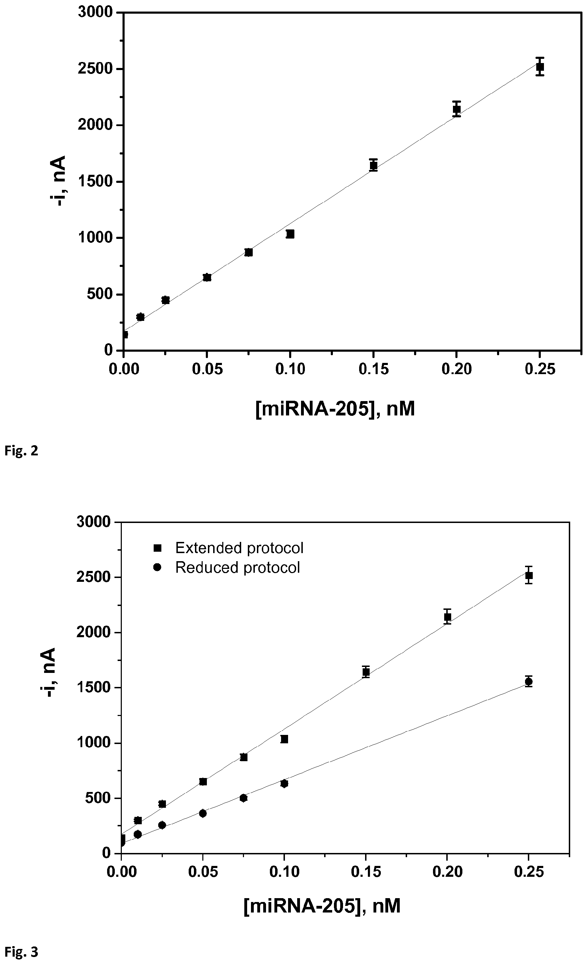

**FIG 2****.** Calibration plot obtained with the developed biosensor using the extended protocol for synthetic target miRNA-205. Error bars were calculated as the triple of the standard deviation (n = 3).
**FIG 3****.** Comparison of the calibration plots obtained with biosensors developed using extended and reduced protocols for synthetic target miRNA-205. Error bars were calculated as the triple of the standard deviation (n = 3).

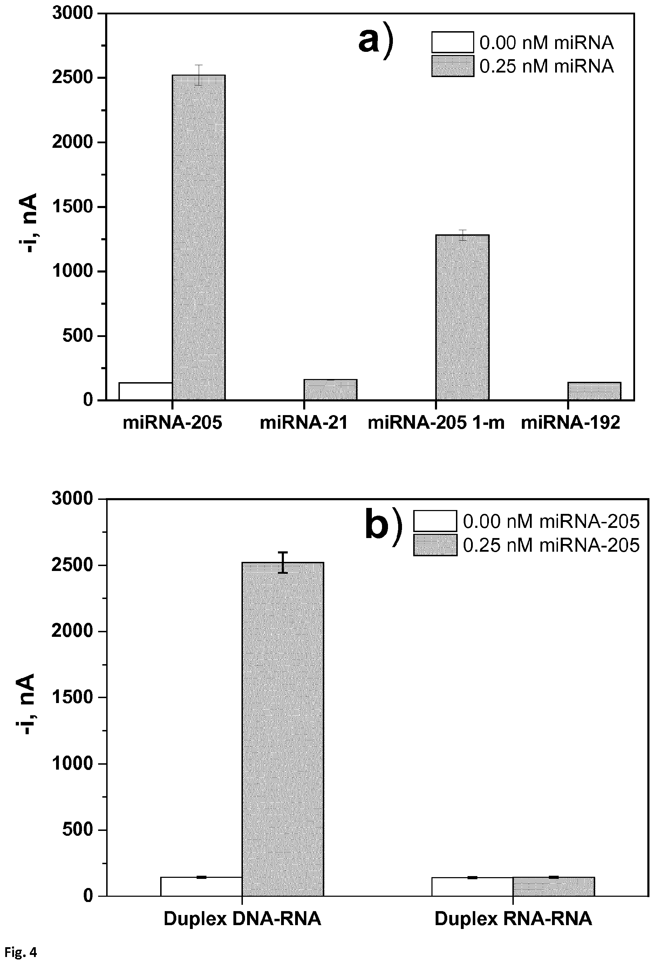

**FIG 4****.** Selectivity tests performed using the extended protocol for synthetic target miRNA-205 using the AbS9.6-MBs based amperometric biosensor. Amperometric responses obtained in the absence or in the presence of 0.25 nM of synthetic target miRNA-205, miRNA-205 1-m and NC sequences (miRNA-21 and miRNA-192) (a). Amperometric responses obtained in the absence and in the presence of 0.25 nM of synthetic target miRNA-205 by performing the homogeneous hybridization with the antiDNA-205 (Duplex DNA/RNA) and the antimiRNA-205 probes (Duplex RNA/RNA) (b).

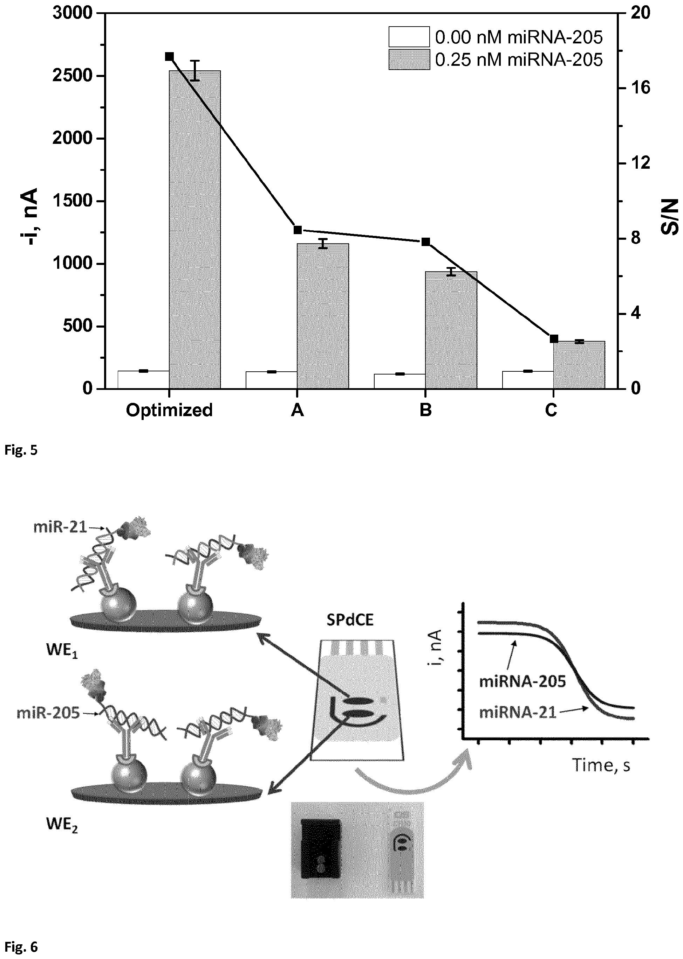

**Fig. 5****.** Amperometric responses measured at -0.20 V and the corresponding S/N ratios obtained for 0 (N, white bars) and 0.25 nM (S, grey bars) synthetic target miRNA-205 as a function of the number of steps involved in the experimental protocol (see Table XII for details). Error bars were estimated as triple of the standard deviation (n=3).

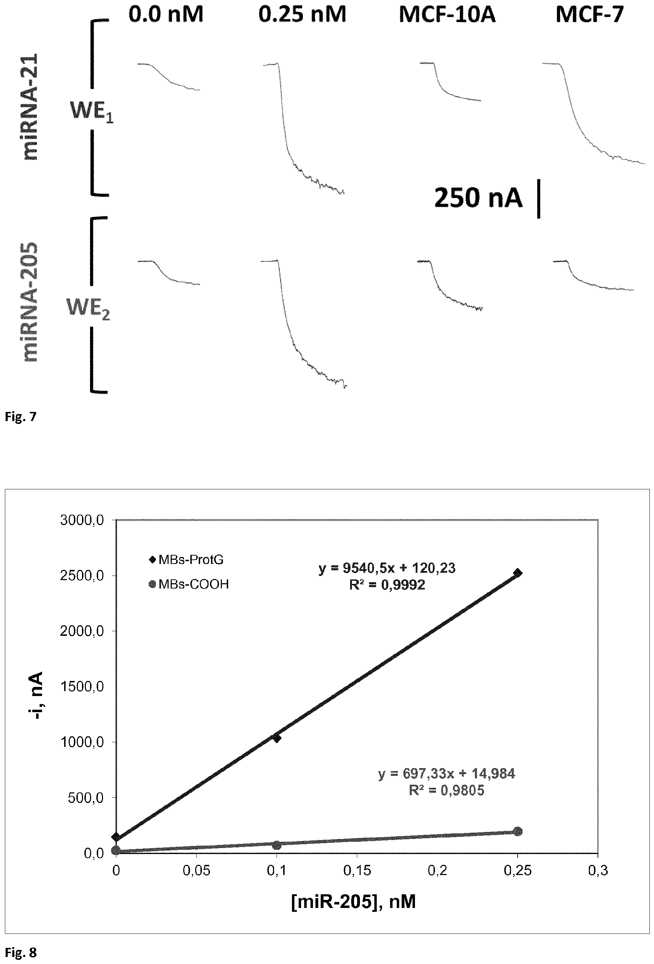

**Fig. 6****.** Schematic display of the fundamentals involved in the dual determination of miRNA-21 and miRNA-205 using the present invention. Real pictures of the dual SPCE and the homemade magnetic holding block. The working variables used were the same than those described for the individual detection of one miRNA. Figure 7 shows the amperometric measurements obtained in the dual determination of miRNA-21 and miRNA-205 in the absence and in the presence of 0.25 nM of the synthetic target miRs and in the presence of 500 ng of RNAₜ extracted from MCF-10A and MCF-7.
**Fig. 7****.** Simultaneous amperometric responses measured at dual SPCEs for mixtures containing: 0 nM and 0.25 nM of both synthetic miRNAs and 500 ng of RNAₜ extracted from MCF-10A and MCF-7 cells.
Eₐₚₚ=-0.20 V vs. Ag pseudo-reference electrode. These results demonstrate the viability of the methodology described in this invention also for the simultaneous detection of multiple miRNAs through a combination of multiplexed detection and different DNA probes. Although this example is focused only on two specific miRNAs, the methodology presented in this invention could be easily extended to the simultaneous profiling of multiple miRNAs.
**Fig. 8****.** Comparison of the calibration curves obtained for miRNA-205 determination with electrochemical platforms based on the use of AbS9.6 immobilized onto HOOC-MBs (in red) and ProtG-MBs (in blue, electrochemical platform of the invention). (Error bars estimated as triple of the standard deviation (n=3)).
**Fig. 9****.** Amperometric responses obtained with the electrochemical platform of the invention for mature miRNA-21 (left) and miRNA-205 (right) in 1.0 g raw RNAₜ extracted from FFPE human breast tissues. Amperometric traces recorded with the electrochemical biosensor for miRNA-21 (left) and miRNA-205 (right) in representative samples. (Error bars estimated as triple of the standard deviation (n=3)).
**Fig. 10****.** The figure provides mean values and standard errors for both biomarkers in the three different HER2 subtypes.
**Fig. 11****.** This figure provides mean concentration of target mature miRNAs (expressed in amol per ng of RNAₜ) in FFPE breast tissues.

### DETAILED DESCRIPTION OF THE INVENTION

### DEFINITIONS

As used herein, the term "electrochemical biosensor based on the use of magnetic beads" or "magnetic beads-based electrochemical biosensor" is understood as a device based on the appropriate coupling of modified magnetic beads and an electrochemical support.

As used herein the term magnetic particles (MBs) refers to nano- to micro-magnetic particles (φ = 10 nm-10 µm) with magnetic properties, being superparamagnetic or not modified with any functional group which allows the immobilization of biomolecules onto their surface. Particularly "superparamagnetic particles (MBs)" as used in this invention are uniform, 2.8 µm, superparamagnetic beads with recombinant Protein G (approx. 17 kDa) coupled to the surface. The beads are supplied at a concentration of 30 mg/ml in phosphate buffered saline (PBS), pH 7.4, containing 0.01% Tween®-20 and 0.09% sodium azide (NaN₃).

As used herein, the term electrochemical support is understood as any rigid or flexible support which can function as a working electrode in an electrochemical transduction.

As used herein, the term "AbS9.6" or "S9.6" is understood as a mouse monoclonal antibody, commercialized by Kerafast and generated against a φX174 bacteriophage-derived synthetic DNA-RNA antigen which recognizes selectively RNA-DNA hybrids of various lengths. The ATCC reference for this monoclonal antibody is S9.6 (ATCC® HB-8730™).
As used herein, the term "antipoly(A)-poly-(dt)" is understood as antibodies specific to RNA-DNA hybrids produced by animal immunization with poly(A).poly(dT).

As used herein, the term "antibodies able to recognize DNA-DNA duplexes" or "antibodies able to recognize RNA-RNA duplexes" is understood as any antibody or a fragment thereof capable of recognizing said duplexes with affinities constants in the range of 10⁵ -10¹² mol⁻¹.

As used herein, the term "homogenous solution" is understood as an in solution (liquid) phase hybridization process, in which both the probe and the target are both freely mobile in solution.

As used herein, a complex capable of being detected and/or quantified by a magnetic beads-based electrochemical sensor is understood as a complex capable of producing an electrochemical reaction by itself or after reaction with other molecules.

As used herein, the term "compound A" is understood as any compound of low molecular weight used to label biomolecules and give an electrochemical signal by itself, like any redox label such as (but not restricted to) ferrocene, methylene blue or inorganic nanocrystals (CdS, ZnS, PbS), or after binding with another compound, such as (but not restricted to) biotin, streptavidin, avidin, digoxigenine and fluorescein.

As used herein the term "RNA/DNA hybrid", "DNA/DNA hybrid", "RNA/RNA hybrid" or "RNA-DNA heteroduplexes" is understood as duplex formed by hybridization of two sequences fully complementary.

As used herein, the term "suspension" is understood as a mixture of liquids with magnetic beads which may not dissolve in the liquid and may be separated from the liquid by leaving it to stand or magnetization.

### DETAILED DESCRIPTION

As an initial matter, the present disclosure is generally directed to methods for detecting and/or quantifying miRNA, RNA or DNA molecules of interest in at least one isolated biological sample. Basically the general procedure followed by the methods of the present invention are illustrated in figure 1 and involve four main steps: (i) homogeneous hybridization of the synthetic DNA or RNA probe and the target miRNA, RNA or DNA molecule of interest; (ii) capture of the resultant DNA/RNA, DNA/DNA or RNA/RNA duplex by the antibody-modified magnetic particles, preferably MBs, in solution; (iii) enzymatic labeling of the biotinylated DNA/RNA, DNA/DNA or RNA/RNA duplex captured onto the antibody-modified magnetic particles, preferably MBs and (iv) electrochemical detection of the modified-magnetic particles on an electrochemical sensor, preferably on screen-printed carbon electrodes (SPCEs). By using this procedure, up to 30 sensors a day can be worked (or more if the process is automated), illustrating the use of the present methodology for the rapid, simple, specific, quantitative and ultrasensitive detection and identification (detection limit [LOD] = 2.4 pM) of polynucleotides (endogenously single-stranded or previously denatured if naturally double-stranded) such as the miRNAs present in the given sample.

The detection limits obtained are illustrated in the examples below, showing the great sensitivity of the sensors of the present disclosure as devices with relevant usefulness in the analysis of complex samples, in particular in the analysis of miRNAs in clinical samples. In the present invention and as shown thought-out the present document, the presence of other DNA or RNA molecules such as miRNAs in the analyzed sample did not interfere with the detection and identification of the miRNA illustrated herein, this is crucial in the context of clinical diagnosis as discussed below.

In order to carry out the present invention, the authors first developed superparamagnetic particles (MBs) labeled with an antibody or a fragment thereof capable of recognizing said RNA-DNA heteroduplexes. For this purpose the antibody AbS9.6 capable of recognizing RNA-DNA heteroduplexes was immobilized through its Fc region to ProtG-functionalized magnetic beads (by incubating 2.5 µL of ProtG-MBs with 25 µL of a 2.0 µg mL⁻¹ AbS9.6 solution prepared in PBS, pH 7.5, for 45 min at 37 °C and 950 rpm). Thereafter, the authors performed a hybridization between the target DNA or RNA molecule and a biotinylated anti-DNA or anti-RNA probe in homogeneous solution. Secondly the previously produced antibody AbS9.6 capable of recognizing RNA-DNA heteroduplexes was used as capture receptor for the DNA/miRNA duplex formed previously in solution. Subsequently, the attached biotinylated hybrid was labeled with a commercial Strep-HRP polymer. To perform the electrochemical transduction, the modified MBs (re-suspended in 45 µL of 0.05 M sodium phosphate buffer solution, pH 6.0) were then magnetically captured on a solid support comprising a solid electrode made of carbon, and a neodymium magnet. After magnetic capturing the resulting MB in the electrodes, the detection of the hybridization process was performed by amperometry using the system hydroquinone (HQ) as redox mediator and H₂O₂, as enzymatic substrate. The added hydrogen peroxide was reduced in the presence of HRP, and the regeneration of the reduced form of the enzyme was mediated by HQ_{red} (Martin et al., 2014. J. Mater. Chem. B, 2: 739-746). The HQₒₓ generated is electrochemically reduced when the potential applied is more negative than the formal potential of the redox HQ_{red}/HQₒₓ pair.

As shown in example 3.1, by using the procedure detailed above and the protocol shown in table I, the authors were able to detect and quantify miRNA-205 in a total assay time of approx. 75 min. In addition, the operational and analytical characteristics of this methodology are summarized in table II, wherein the calibration graph constructed for the synthetic miRNA-205 target under the optimized experimental conditions (displayed in Figure 2) showed linearity (r=0.998) between 8.2 and 250 pM, with a sensitivity of (9548 ± 211) nA nM⁻¹ and a limit of detection (LOD, estimated as 3×s_{b}/m, in which s_{b} is the standard deviation of the blank, and m is the slope of the calibration curve) of 2.4 pM (6 attomoles in 25 µL of sample), without the use of any amplification or pre-concentration technique.

In addition, as shown in example 3.2 by slightly changing the protocol shown in example 3.1, the total assay time was reduced from approx. 75 min to approx. 30 min. Figure 3 shows a comparison between the extended and reduced protocols, said comparison is also illustrated in table IV.

Therefore, the methodology illustrated in the present invention provides for a reliable assay capable of providing results in approximately 30 minutes. This fact highlights the usefulness of the present methodology in the analysis of clinical samples, in particular in the analysis of miRNAs in clinical samples, wherein a rapid, sensitive and reliable method is especially useful. In addition, the reliability of the present methodology is further illustrated in example 4 wherein the selectivity of the methodology developed, was evaluated by comparing the amperometric signal obtained with electrochemical sensors fabricated using the extended protocol as shown in example 3.1 in the absence of target miRNA and in the presence of 0.5 nM of a fully complementary sequence (miRNA-205) of the DNA probe using in the assay (antiDNA-205), a single mismatched base (miRNA-205 1-m, sequence in Table 1) and 2 completely non-complementary sequences (miRNA-21 and miRNA-192, sequences in Table 1).

As it can be deduced from the results displayed in Figure 4a), the sensor responses to the non-complementary sequences were not significantly different from that measured in the absence of target miRNA. Moreover, the 1-m sequence provided 51 % of the response to the target miRNA-205. At this point, it is important to remark that the probability of finding in the same sample the complementary sequence and an equivalent with a single mismatched base is very low (Labib et al., J. Am. Chem. Soc. 135 (2013) 3027-3038.). Moreover, results presented in Figure 4b) demonstrated the high affinity of the AbS9.6 only for DNA/RNA hybrids. These results clearly indicate the high selectivity of the methodology developed.

As regards the reproducibility of the methodology developed, example 5 illustrates the great reliability in the whole procedure involving both the sensor fabrication protocol (MBs modification and magnetic capture on the SPCE surface) and the amperometric transduction.

Furthermore, the methodology developed is also highly versatile as clearly reflected in example 7, wherein the results shown therein illustrate that the present methodology is applicable to any miRNA, RNA or DNA single stranded or double stranded molecule of interest present in an isolated biological sample.

Lastly, taking into account the interest in the multiplexed determination of miRNAs in clinical samples we also evaluated the potential of the methodology developed in this invention to simultaneously detect the expression of two different miRNAs in one single experiment at dual SPCEs. The fundamentals of the simultaneous determination of miR-21 and miR-205 using the disposable dual electrochemical magnetic beads-based biosensor are displayed in Figure 6 and explained in example 10. As illustrated therein the present methodology is a remarkably useful tool for the multiplexed determination of miRNA, RNA or DNA single stranded or double stranded molecules in complex clinical samples.

Consequently and based on these results, the methodology developed by the authors of the present invention for detecting and/or quantifying miRNA, RNA or DNA molecules of interest in at least one isolated biological sample, provides for a reliable methodology capable of rapidly (in approx. less than 75 min, preferably in about 30 minutes) providing reproducible results with high specificity and sensitivity in complex clinical samples of different types of specimens. The fact that the present methodology is especially useful in complex clinical samples is illustrated in examples 8 and 10 wherein as summarized in tables IX and X the results provided by the magnetosensor showed over-expression of miRNA-21 and reduced expression of miRNA-205 in the metastatic breast cell line (MCF-7) compared to the normal-like MCF-10A cell line, which was in good agreement with that reported earlier (Ribas et al., Trends in Anal. Chem. 49 (2013) 20-30), (Campuzano et al., Biosens. Bioelectron. 66 (2015) 385-391), (Elgamal et al., PLos ONE 8(10) 2013: e76402. doi:10.1371/journal.pone.0076402).

Very interesting results were also obtained by applying the developed sensor to the analysis of miRNA-21 in total RNA (RNAt) extracted from human serum samples, where miRNA-21 was highly expressed only in samples from breast cancer patients compared to those form healthy women.

Therefore, a first aspect of the disclosure refers to a method to detect and/or quantify miRNA, RNA or DNA molecules of interest in at least one isolated sample, preferably a biological sample, through an electrochemical sensor based on the use of magnetic beads (magnetic beads-based electrochemical sensor), comprising or consisting of the following sequential steps:
a. Obtaining a RNA/DNA, DNA/DNA or RNA/RNA hybrid by carrying out a homogenous hybridization in solution of a miRNA, RNA or DNA molecule of interest present in the isolated sample to a complementary RNA or DNA sequence labeled with a compound A, to provide the RNA/DNA, DNA/DNA or RNA/RNA hybrid;
b. Capturing the product of the hybridization in solution of step a) by adding to said solution a suspension of magnetic particles, preferably superparamagnetic particles (MBs), previously bound to an antibody or a fragment thereof capable of recognizing RNA-DNA heteroduplexes or DNA-DNA or RNA-RNA duplexes, wherein said product of the hybridization is captured onto the said magnetic particles;
c. Adding an element capable of being detected and/or quantified by a magnetic beads-based electrochemical sensor, wherein said element binds to compound A, thereby generating a complex capable of being detected and/or quantified by a magnetic beads-based electrochemical sensor;
d. Magnetic capturing the product of step c) comprising the magnetic particles onto an electrochemical support; and
e. detecting and/or quantifying a electrochemical signal produced by the complex of step d), thereby detecting and/or quantifying the product of hybridization of step a) and thereby detecting and/or quantifying the miRNA, RNA or DNA molecules of interest present in the at least one sample, preferably a biological sample.

It is important to note that the order of the steps provided in the method of the first aspect of the disclosure is not casual as shown in example 9. In this sense and as illustrated in said example, different protocols (described in detail in table XII) were assayed to test the interaction between the AbS9.6 and the DNA/miRNA duplex. Figure 5 shows the amperometric responses measured for 0 and 0.25 nM of synthetic target miRNA-205 as well as the corresponding calculated S/N ratios. As it can be seen, although it is possible to detect the presence of the target miRNA using all the protocols, the largest S/N ratio was achieved when the AbS9.6 was used immobilized on the surface of MBs and when the labeling of the DNA/RNA duplex is performed when the duplex was previously captured by the AbS9.6-MBs. These results can be attributed to a poorer immobilization of the AbS9.6 onto the MBs after duplex recognition and to a hindered recognition of the DNA/RNA duplex previously labeled with the Strep-HRP polymer by the AbS9.6-MBs. Consequently, the 4-step protocol, involving selective recognition of the DNA/RNA duplex formed previously in solution by the pre-modified magnetic particles and the final enzymatic labeling of the duplex captured onto the pre-modified magnetic particles, was selected in order to achieve the best sensitivity.

In any case, as already stated, it is possible to detect the presence of the target miRNA using all the protocols shown although these are significantly less sensitive than the optimized method of the present invention. Therefore, an alternative embodiment of the first aspect of the disclosure refers to a method to detect and/or quantify miRNA, RNA or DNA molecules of interest in at least one isolated sample, preferably a biological sample, through an electrochemical sensor based on the use of magnetic beads (magnetic beads-based electrochemical sensor), comprising or consisting of the following sequential steps:
a. Obtaining a RNA/DNA, DNA/DNA or RNA/RNA hybrid by carrying out a homogenous hybridization in solution of a miRNA, RNA or DNA molecule of interest present in the isolated sample to a complementary RNA or DNA sequence labeled with a compound A, to provide the RNA/DNA, DNA/DNA or RNA/RNA hybrid;
b. Forming a complex with the product of the hybridization in solution of step a) by adding to said solution a suspension comprising an antibody or a fragment thereof capable of recognizing RNA-DNA heteroduplexes or DNA-DNA or RNA-RNA duplexes;
c. Capturing the complex of step b), in particular the antibody or fragment thereof binding the RNA-DNA heteroduplexes or DNA-DNA or RNA-RNA duplexes, by binding these complexes onto magnetic particles, preferably superparamagnetic particles (MBs);
d. Adding an element to the product of step c) capable of being detected and/or quantified by a magnetic beads-based electrochemical sensor, wherein said element binds to compound A thereby generating a complex capable of being detected and/or quantified by a magnetic beads-based electrochemical sensor;
e. Magnetic capturing the product of step d) comprising the magnetic particles onto an electrochemical support; and
f. detecting and/or quantifying a electrochemical signal produced by the complex of step d), thereby detecting and/or quantifying the product of hybridization of step a) and thereby detecting and/or quantifying the miRNA, RNA or DNA molecules of interest present in the at least one sample, preferably a biological sample.

A further alternative embodiment of the first aspect of the disclosure refers to a method to detect and/or quantify miRNA, RNA or DNA molecules of interest in at least one isolated sample, preferably a biological sample, through an electrochemical sensor based on the use of magnetic beads (magnetic beads-based electrochemical sensor), comprising or consisting of the following sequential steps:
a. Obtaining a RNA/DNA, DNA/DNA or RNA/RNA hybrid by carrying out a homogenous hybridization in solution of a miRNA, RNA or DNA molecule of interest present in the isolated sample to a complementary RNA or DNA sequence labeled with a compound A, to provide the RNA/DNA, DNA/DNA or RNA/RNA hybrid and forming a complex with the product of the hybridization by simultaneously to the addition of the complementary RNA or DNA sequence adding to said solution a suspension comprising an antibody or a fragment thereof capable of recognizing RNA-DNA heteroduplexes or DNA-DNA or RNA-RNA duplexes;
b. Capturing the complex of step a), in particular the antibody or fragment thereof binding the RNA-DNA heteroduplexes or DNA-DNA or RNA-RNA duplexes, by binding these complexes onto magnetic particles, preferably superparamagnetic particles (MBs);
c. Adding an element to the product of step b) capable of being detected and/or quantified by a magnetic beads-based electrochemical sensor, wherein said element binds to compound A thereby generating a complex capable of being detected and/or quantified by a magnetic beads-based electrochemical sensor;
d. Magnetic capturing the product of step c) comprising the magnetic particles onto an electrochemical support; and
e. detecting and/or quantifying a electrochemical signal produced by the complex of step d), thereby detecting and/or quantifying the product of hybridization of step a) and thereby detecting and/or quantifying the miRNA, RNA or DNA molecules of interest present in the at least one sample, preferably a biological sample.

A still further alternative embodiment of the first aspect of the disclosure refers to a method to detect and/or quantify miRNA, RNA or DNA molecules of interest in at least one isolated sample, preferably a biological sample, through an electrochemical sensor based on the use of magnetic beads (magnetic beads-based electrochemical sensor), comprising or consisting of the following sequential steps:
a. Obtaining a RNA/DNA, DNA/DNA or RNA/RNA hybrid by carrying out a homogenous hybridization in solution of a miRNA, RNA or DNA molecule of interest present in the isolated sample to a complementary RNA or DNA sequence labeled with a compound A, to provide the RNA/DNA, DNA/DNA or RNA/RNA hybrid;
b. Adding an element capable of being detected and/or quantified by a magnetic beads-based electrochemical sensor to the solution of step a), wherein said element binds to compound A thereby generating a complex capable of being detected and/or quantified by a magnetic beads-based electrochemical sensor;
c. Capturing the product in solution of step b) by adding to said solution a suspension of magnetic particles, preferably superparamagnetic particles (MBs), previously bound to an antibody or a fragment thereof capable of recognizing RNA-DNA heteroduplexes or DNA-DNA or RNA-RNA duplexes, wherein said product of the hybridization of step b) is captured onto the said magnetic particles;
d. Magnetic capturing the product of step c) comprising the magnetic particles onto an electrochemical support; and
e. detecting and/or quantifying a electrochemical signal produced by the complex of step d), thereby detecting and/or quantifying the product of hybridization of step a) and thereby detecting and/or quantifying the miRNA, RNA or DNA molecules of interest present in the at least one sample, preferably a biological sample.

In addition, it is further noted that the suspension of magnetic particles mentioned in any of the above methods, preferably superparamagnetic particles (MBs), is previously bounded to an antibody or a fragment thereof capable of recognizing RNA-DNA heteroduplexes or DNA-DNA or RNA-RNA duplexes through protein G coupled to the surface of the magnetic particles. In this sense by using magnetic particles coupled to or modified with protein G, the complex magnetic particles-antibody provides for sufficient electrochemical signal intensity as to provide a method capable of reliably quantifying miRNA, RNA or DNA molecules in biological samples such as complex clinical samples. In order to demonstrate this point, we have performed a series of experiments reproducing the methodology illustrated in the optimized protocol of example 9 by using ProteinG-MBs and by using MBs-COOH, the results are illustrated below and in figure 8.

**ProteinG-MBs**

| [miR-205], nM | signal, nA |
|---|---|
| 0 | 143.7 |
| 0.1 | 1035.2 |
| 0.25 | 2521.0 |

**MBs-COOH**

| [miR-205], nM | signal, nA |
|---|---|
| 0 | 23.5 |
| 0.1 | 70.5 |
| 0.25 | 195.0 |

As reflected above, by using protein G coated or modified MBs the results provide a sufficiently sensitive and quantifiable methodology. The sensitivity 14 times higher achieved onto ProteinG-MBs can be attributed to the claimed orientation controlled by the bacterial proteinG because of its selective attachment to the Fc region of the antibodies. It is noted that Protein G (MW17kDa) is a cell surface protein of group C and G Streptococci with three Fc binding domains located near its C-terminal and exhibits specificity for subclasses of antibodies from many species. Therefore, the oriented binding of antibodies through their Fc region using ProteinG-MBs leaves their Fab region exposed to the solution and free to bind the antigen thus providing immunosensors with significantly improved performance compared to those based on non-Fc region oriented immobilization approaches.

In addition, the methodology illustrated above provides the capability to perform accurate miRNA determination in FFPE (formalin-fixed, paraffin-embedded) tissues sections, which is of great interest considering the multiple benefits of using FFPE tissue samples in clinical research for the diagnosis and therapeutic follow-up of a wide range of cancers and other human pathologies (inflammation, immune related diseases, etc.). FFPE is the standard method of tissue processing used in Pathology Departments for cancer diagnosis. Working with FFPE samples opens up to a huge potential of novel candidate miRNA as diagnostic or prognostic cancer biomarkers.

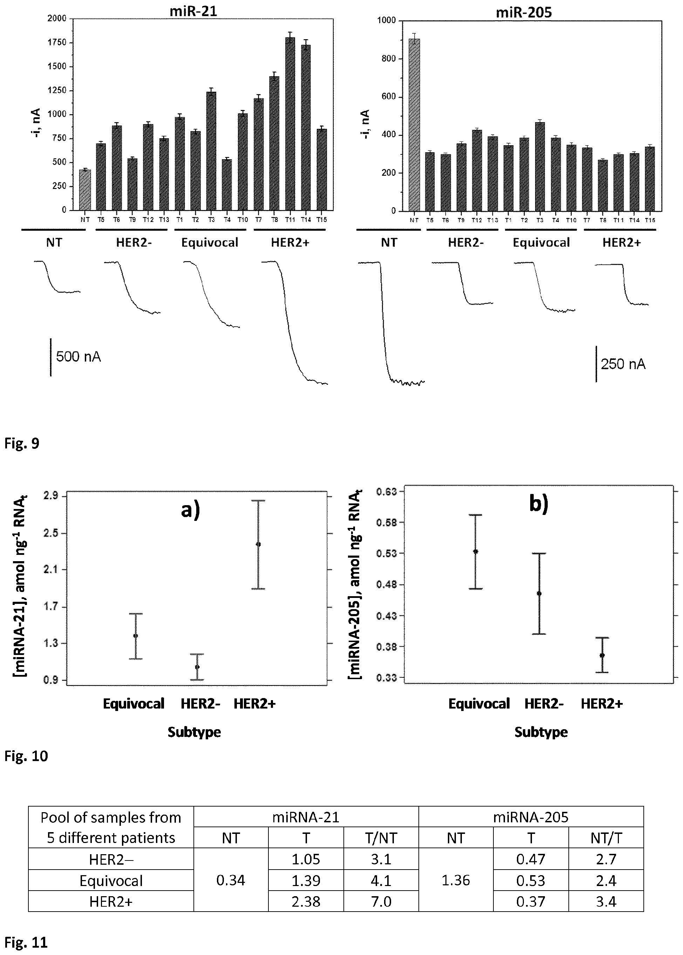

There is a vast amount of FFPE tissue samples housed in hospitals, clinics, and research facilities, available to be studied which would allow a quicker advance research in disease diagnostics, outcomes, and therapies. In this sense, the present specification offers a proof of concept, to outline the feasibility and potential applicability of this recently developed electrochemical approach to perform quantitative determination of any target miRNA in FFPE tissues. Moreover, the use of simple, portable and cost-effective instrumentation suitable to perform multiplexed detection required by electrochemical transduction, can be claimed as an important practical advantage in the implementation of user-friendly and affordable devices to perform routine and decentralized analysis. The results in FFPE tissue samples by using the methodology illustrated in the optimized protocol of example 9 are illustrated herein in figures 9 to 11.

Consequently, it is thus clear the feasibility and potential applicability of the developed electrochemical approach to perform quantitative determination of any target miRNA in FFPE tissues with a similar sensitivity and accuracy as that obtained with fresh-frozen samples.

It is noted that the term "compound A" is understood in the present disclosure as any compound of low molecular weight used to label biomolecules and give an electrochemical signal by itself, like any redox label such as (but not restricted to) ferrocene, methylene blue or inorganic nanocrystals (CdS, ZnS, PbS), or after binding with another compound, such as (but not restricted to) biotin, streptavidin, avidin, digoxigenine and fluorescein. In this sense, it is noted that the previously mentioned methods are all restricted to the use of a compound A understood as a compound capable of providing an electrochemical signal after binding with another compound, such as (but not limited to) biotin, streptavidin, avidin, digoxigenine and fluorescein. However, the present disclosure also encompasses the use of a compound A understood as a compound capable of providing an electrochemical signal by itself, like any redox label such as (but not limited to) ferrocene, methylene blue or inorganic nanocrystals (CdS, ZnS, PbS). Therefore, a further alternative embodiment of the first aspect of the disclosure refers to any of the previously mentioned methods of the invention wherein compound A is understood as a compound capable of providing an electrochemical signal by itself and wherein the step of adding an element capable of being detected and/or quantified by a magnetic beads-based electrochemical sensor, wherein said element binds to compound A thereby generating a complex capable of being detected and/or quantified by a magnetic beads-based electrochemical sensor, is omitted since it is no longer necessary.

In a preferred embodiment of the first aspect of the disclosure or of any of its alternative or preferred embodiments, the antibody or fragment thereof is selected from the list consisting of antipoly(A)-poly-(dt) and S9.6.

In another preferred embodiment of the first aspect of the disclosure or of any of its alternative or preferred embodiments, the molecule of interest is a miRNA molecule, more preferably any of miRNA-205, miRNA-21, miR-223, miRNA-26b5p, miRNA-27a3p, miRNA-1273p and miRNA-933p.

In another preferred embodiment of the first aspect of the disclosure or of any of its alternative or preferred embodiments, compound A is a compound capable of binding with a compound selected from the group consisting of biotin, streptavidin, avidin, digoxigenin and fluorescein.

In another preferred embodiment of the first aspect of the disclosure or of any of its alternative or preferred embodiments, the element capable of being detected and/or quantified by a magnetic beads-based electrochemical biosensor is a complex formed by an element capable of binding to compound A and an enzyme capable of being detected and/or quantified by a magnetic beads-based electrochemical sensor. More preferably, said enzyme is an oxidase or a hydrolase such as phosphatase alkaline, preferably a peroxidase, more preferably the horseradish peroxidase (HRP) enzyme, and preferably said detection and/or quantification is carried out by monitoring the enzymatic reduction of an enzymatic substrate system (substrate and co-substrate), preferably H₂O₂ and hydroquinone.

In another preferred embodiment of the first aspect of the disclosure or of any of its alternative or preferred embodiments, the isolated sample is a biological sample, preferably a tissue sample or a biological fluid such as blood, serum, plasma, cerebrospinal fluid, saliva or urine.

In yet another preferred embodiment of the first aspect of the disclosure or of any of its alternative or preferred embodiments, the electrochemical sensor comprises i) a solid electrode made of a material selected from the list consisting of: gold, carbon, platinum, CD-trode, screen-printed electrodes, silver, mercury, graphite, glassy carbon, carbon nanotubes, gold nanowires, gold nanoparticles, metallic oxide nanoparticles, carbon paste, boron-doped diamond and composites, and ii) a magnet, preferably a neodymium magnet.

In still another preferred embodiment of the first aspect of the disclosure or of any of its alternative or preferred embodiments, the fragment thereof is selected from the group consisting of "Fab" fragments, "F(ab')2" fragments, "Fv" fragments, single chain Fv fragments or "scFv", "Diabodies" and "bispecific antibodies" (Bab).

A second aspect of the disclosure refers to an antibody or a fragment thereof selected from the group consisting of "Fab" fragments, "F(ab')2" fragments, "Fv" fragments, single chain Fv fragments or "scFv", "Diabodies" and "bispecific antibodies" (Bab), wherein said antibody or fragment thereof is bound to magnetic particles, preferably superparamagnetic particles (MBs), and wherein said antibody or fragment thereof is capable of recognizing RNA-DNA heteroduplexes or DNA-DNA or RNA-RNA duplexes.

Preferably said antibody or fragment thereof is found in a composition, preferably in the form of a suspension.

It is noted that the modified magnetic particles of the second aspect of the disclosure are especially useful in the optimized methodology illustrated in example 9 of the present invention. In addition and also in connection to the second aspect of the disclosure, it is important to highlight that the conjugated magnetic particles, preferably superparamagnetic particles (MBs), do not solely constitute an essential element for the optimized methodology discussed in the first aspect of the disclosure but it also possesses a remarkable stability which makes it especially useful to carry out the present teaching in light of the fact that these modified particles must be produced before implementing the aforesaid methodology. Example 6 demonstrates such feature (the stability); by describing an assay in which AbS9.6-MBs were prepared and stored at 4 °C in eppendorf tubes containing 50 µL of filtered PBS. Each working day one batch of the prepared conjugates was incubated in a solution containing 0.075 nM of the synthetic target miRNA-205 according to the extended protocol. A control chart was constructed by setting the average current value calculated from 5 measurements made the first day of the study (day of the AbS9.6-MBs preparation) as the central value, while the upper and lower limits of control were set at ±3×SD of these initial values. The obtained results (shown in Table VII) showed that the biosensor responses remained within the control limits for at least 29 days (no longer times were assayed). This good storage stability suggests the possibility of preparing sets of AbS9.6-MBs conjugates and storing them under the above mentioned conditions until their use for the biosensor preparation is required.

A third aspect of the disclosure refers to the use *in vitro* of the antibody or fragment thereof of the second aspect of the disclosure or of the composition also described in said aspect of the disclosure for detecting and/or quantifying miRNAs, RNA or DNA molecules of interest in at least one isolated sample, preferably a biological sample, by using a magnetic beads-based electrochemical biosensor, preferably for implementing any of the methods, preferably the optimized method, as defined in the first aspect of the disclosure or in any of its preferred embodiments.

A fourth aspect of the disclosure refers to a kit or device for detecting and/or quantifying miRNAs, RNA or DNA molecules of interest in at least one sample, preferably an isolated biological sample, by using a magnetic beads-based electrochemical biosensor, which comprises:
a. the antibody or fragment thereof of the second aspect of the invention or the composition also described in said aspect of the invention;
b. a RNA or DNA molecule labeled with a compound A complementary to the miRNA, RNA or DNA molecule of interest;
c. optionally an element capable of being detected and/or quantified by a magnetic beads-based electrochemical biosensor, wherein said element binds to compound A; and
d. optionally a solution of the enzymatic substrate system (substrate and co-substrate), preferably H₂O₂ and hydroquinone.

In a preferred embodiment of the fourth aspect of the disclosure, said biosensor further comprises a support which in turn comprises i) a solid electrode made of a material selected from the list consisting of: gold, carbon, platinum, CD-trode, screen-printed electrodes, silver, mercury, graphite, glassy carbon, carbon nanotubes, gold nanowires, gold nanoparticles, metallic oxide nanoparticles, carbon paste, boron-doped diamond and composites, and ii) a magnet, preferably a neodymium magnet. Preferably, said solid electrode is made of carbon, wherein the antibody or fragment thereof capable of recognizing RNA-DNA heteroduplexes or the DNA-DNA or RNA-RNA duplexes is selected from the list consisting of antipoly(A)-poly-(dt) and S9.6 and wherein the molecule of interest is a miRNA.

Furthermore, a fifth aspect of the disclosure refers to the use *in vitro* of the kit or device of the fourth aspect of the disclosure, for detecting and/or quantifying miRNAs, RNA or DNA molecules of interest in at least one isolated sample, preferably a biological sample, by using an electrochemical signal; preferably for use in implementing any of the methods, preferably the optimized method, as defined in the first aspect of the disclosure or in any of its preferred embodiments.

Finally, the present disclosure can be further implemented in a microfluidic device capable of moving and capturing magnetic beads and performing electrochemical detection in a series of electrodes. This microfluidic chip will integrate a series of channel microband electrodes, and a capture zone in which functionalized magnetic particles are captured. The disposable chip will be of the size of a microscope slide and contains independent measuring units. This allows the simultaneous or sequential performance of multiple microRNA measurements on the same chip. A holder to host the chip and provide electrical and fluidic connections will be fabricated using 3D printing techniques.

By "comprising" it is meant including, but not limited to, whatever follows the word "comprising". Thus, use of the term "comprising" indicates that the listed elements are required or mandatory, but that other elements are optional and may or may not be present.

By "consisting of" is meant including, and limited to, whatever follows the phrase consisting of. Thus, the phrase "consisting of" indicates that the listed elements are required or mandatory, and that no other elements may be present.

The inventions illustratively described herein may suitably be practiced in the absence of any element or elements, limitation or limitations, not specifically disclosed herein. Thus, for example, the terms "comprising", "including", "containing", etc. shall be read expansively and without limitation. Additionally, the terms and expressions employed herein have been used as terms of description and not of limitation, and there is no intention in the use of such terms and expressions of excluding any equivalents of the features shown and described or portions thereof, but it is recognized that various modifications are possible within the scope of the invention claimed. Thus, it should be understood that although the present invention has been specifically disclosed by preferred embodiments and optional features, modification and variation of the inventions embodied therein herein disclosed may be resorted to by those skilled in the art.

The invention has been described broadly and generically herein. Each of the narrower species and subgeneric groupings falling within the generic disclosure also form part of the invention. This includes the generic description of the invention with a proviso or negative limitation removing any subject matter from the genus, regardless of whether or not the excised material is specifically recited herein.

Other embodiments are within the following claims and non-limiting examples. In addition, where features or aspects of the invention are described in terms of groups, those skilled in the art will recognize that the invention is also thereby described in terms of any individual member or subgroup of members of the group.

### EXAMPLES

### Example 1. Materials and methods

### 1. Devices and electrodes used.

Amperometric measurements were performed with a CHI812B potentiostat (CH Instruments) controlled by software CHI812B. All measurements were carried out at room temperature. The transducers employed were screen-printed carbon electrodes (SPCEs) (DRP-110, DropSens) consisting of a 4-mm diameter carbon working electrode, a carbon counter electrode and an Ag pseudo-reference electrode. A magnetic separator (DynaMag™-2) was purchased from Invitrogen Dynal, and a constant temperature incubator shaker from Ivymen-Comecta was also used.

A Raypa steam sterilizer, biological safety cabinet Telstar Biostar, a temperature freezer New Brunswick Scientific, a refrigerated centrifuge Sigma 1-15K, Forma Scientific Infrared CO₂ Incubator and Stuart SBH130 Analogue Block Heater were also used.

To perform the simultaneous determination of two target miRNAs in one single experiment dual screen-printed carbon electrodes (SPdCEs) (DRP-C1110) consisting of two elliptic carbon working electrodes (6.3 mm² each), a carbon counter electrode and a Ag pseudo-reference electrode were purchased from DropSens. Furthermore, a specific cable connector (ref. DRP-BICAC also from DropSens, S.L.) acted as interface between the SPdCEs and the potentiostat.

### 2. Reagents and solutions.

All the reagents used were of the highest available grade.

Sodium di-hydrogen phosphate, di-sodium hydrogen phosphate, NaCl, KCl and Tween®20 were purchased from Scharlab; hydroquinone (HQ), and hydrogen peroxide (30%, w/v) were purchased from Sigma-Aldrich and bovine serum albumin (BSA Type VH) from Gerbu.

Protein G-modified magnetic beads (ProtG-MBs, ThermoFisher Scientific, Catalog number: 10003D), an anti-DNA-RNA Hybrid [S9.6] Antibody (AbS9.6) from Kerafast and a high sensitivity Strep-HRP conjugate from Roche (ref. 11 089 153 001, 500 U mL⁻¹) were also used.

The following buffer solutions, prepared with Milli-Q water (18 MΩ cm at 25 °C), were used: phosphate-buffered saline (PBS) consisting of 0.01 M phosphate buffer solution containing 137 mM NaCl and 2.7 mM KCI, pH 7.5 (sterilized after their preparation) and phosphate buffer 0.1 M, pH 6.0. A commercial blocker casein solution (a ready-to-use, PBS solution of 1% w/v purified casein) was purchased from Thermo Scientific (Catalog number: 37528).

The DNA and RNA oligonucleotides used, whose sequences are described in Table 1, were purchased from Sigma-Aldrich.

**Table 1.- Oligonucleotides used in this work.**

| Oligonucleotide | Sequence (5'→3') |
|---|---|
| AntimiRNA-205 Probe | 5'-AGACUCCGGUGGAAUGAAGGA-Biotin-3' |
| antiDNA-205 Probe | 5'-AGACTCCGGTGGAATGAAGGA-Biotin-3' |
| Target miRNA-205 | 5'-UCCUUCAUUCCACCGGAGUCU-3' |
| antiDNA-21 Probe | 5'-TCAACATCAGTCTGATAAGCTA-Biotin-3' |
| Target miRNA-21 | 5'-UAGCUUAUCAGACUGAUGU-3' |
| antiDNA-223 Probe | 5'-AACTCAGCTTGTCAAATACACG-Biotin-3' |
| Target miRNA-223 | 5'-CGUGUAUUUGACAAGCUGAGUU-3' |
| AntiDNA-26b5p | 5'-TACCTATCCTGAATTACTTGAA-Biotin-3' |
| Target miRNA-26b5p | 5'-UUCAAGUAAUUCAGGAUAGGUA-3' |
| AntiDNA-27a3p | 5'-TGCGGAACTTAGCCACTGTGAA-Biotin-3' |
| Target miRNA-27a3p | 5'-UUCACAGUGGCUAAGUUCCGCA-3' |
| AntiDNA-1273p | 5'-AGCCAAGCTCAGACGGATCCGA-Biotin-3' |
| Target miRNA-1273p | 5'-UCGGAUCCGUCUGAGCUUGGCU-3' |
| AntiDNA-933p | 5'-CGGGAAGTGCTAGCTCAGCAGT-Biotin-3' |
| Target miRNA-933p | 5'-ACUGCUGAGCUAGCACUUCCCG-3' |
| 1-mismatched miRNA-205 (miRNA-205 1-m) | 5'-UCCUUCAUUACACCGGAGUCU-3' |
| miRNA-192 | 5'-CUGACCUAUGAAUUGACAGCC-3' |

All these oligonucleotides were dissolved in nuclease free water at 100 µM final concentration, aliquoted into smaller volumes and stored at -80 °C.

### 3. Cell cultures and RNAₜ extraction.

One breast cancer cell line (MCF-7) and one non-tumorigenic epithelial cell line (MCF-10A) were selected to check the biosensor performance. MCF-7 cells were grown at 37 °C in a humidified atmosphere containing 5% CO₂. MCF-7 cells were maintained in high-glucose DMEM (Dulbecco's modified Eagle's medium), supplemented with 10% fetal bovine serum, 100 U mL⁻¹ penicillin, 100 µg mL⁻¹ streptomycin and 2.5 mM L-glutamine (GIBCO-Invitrogen, Carlsbad, CA, USA), while MCF-10A cells were cultured in high-glucose DMEM/Ham's Nutrient Mixture F12 (1:1) with 2.5 mM L-glutamine (Gibco), 5% (v/v) horse serum (Gibco), 10 mg mL⁻¹ human insulin (Sigma), 0.5 mg mL⁻¹ hydrocortisone (Sigma), 10 ng mL⁻¹ EGF, and 100 ng mL⁻¹ cholera toxin (QuadraTech Ltd).

RNAₜ was isolated from both cell lines using Tri Reagent (Molecular Research Center, Inc.). Briefly, cells were washed with PBS, scraped off and spun down. The pellet was subjected to Tri Reagent and homogenized for 5 min at room temperature before chloroform extraction. RNAₜ in the upper aqueous phase was then precipitated with isopropylalcohol and washed twice in 70% EtOH. Finally the pellet was dried out in a heating plate during 10 min at 80 °C, dissolved in RNase-free water and stored at -80 °C.

RNAₜ quality and concentration were evaluated by measuring the absorbance at 260, 230 and 280 nm with an ND-1000 spectrophotometer (NanoDrop Technologies, Wilmington, DE, USA). In all cases, the expected 260/280 (∼2.0) and 260/230 (2.0-2.2) ratio values accepted for pure RNA were obtained.

### 4. Serum samples and RNAₜ extraction.

Serum samples (collected from healthy and breast cancer patients) were kept at -20° C and RNAₜ was extracted using a commercial kit (Qiagen, miRNesay Serum/Plasma Kit, ref: 217184).

### 5. FFPE breast tissues and RNAₜ extraction

Breast cancer patients signed the corresponding informed consent and the study had the approval of the University Hospital of Getafe's Ethics Committee. Samples from 15 breast cancer (T) and paired normal adjacent (NT) FFPE tissues were collected at the University Hospital of Getafe (Madrid, Spain). HER2 status of all samples was established by immunohistochemistry (IHC) and subsequent silver-based in situ hybridization (SISH) when needed following ASCO/CAP recommendations. Five of them were classified as Luminal A tumors after showing positive estrogen and progesterone receptor (ER and PR) IHC staining and an equivocal (2/3) immunostaining for HER2 with a subsequent SISH analysis that failed to show HER2 amplification (HER2/CEP17 ratio under 2.0 and average HER2 copy number under 4 signals/cell). These 5 cases are, therefore, HER2-negative, and were designed as the group of HER2-equivocal in our study. Another 5 cases had negative immunostaining for ER and PR as well as for HER2. All 5 cases were considered triple-negative BC and included as HER2-negative group in our study. The remaining 5 cases had variable staining for hormonal receptors but a positive and complete circumferential strong IHC staining for HER2 in more than 10% of tumor cells. These constituted the cases of the HER2-positive study group. For RNA extraction, 5 microtome sections from each paraffin tumor block, 6 micron-thick were placed in eppendorf tubes.

RNAₜ was extracted from 1 eppendorf tube of each of all these FFPE tissues using the microRNA easy FFPE kit (QIAGEN) following the recommended protocol and eluting the RNAₜ with 30 microliters of RNase-free water. RNAₜ quality and concentration were evaluated by measuring the absorbance with an ND-1000 spectrophotometer (NanoDrop Technologies, Wilmington, DE, USA). In all cases, the 260/280 and 260/230 absorbance ratio values obtained assess the purity of RNAₜ extracted.

### 6. Modification of MBs.

25 µL of 0.05 µM of the biotinylated antimiRNA probe and the appropriate amount of the synthetic target or the RNAₜ extracted from the sample under study were mixed in an Eppendorf tube containing PBS, pH 7.5. The hybridization mixture was placed in the thermal shaker at 37 °C and a 950 rpm mixing speed for 45 minutes. A control without target miRNA was run in each hybridization to evaluate the blank signal.

2.5 µL of the ProtG-MBs suspension were transferred to an Eppendorf tube and washed twice with 50 µL of PBS. Between each step the particles were concentrated using a magnet and, after 3 min, the supernatant was discarded. The washed MBs were incubated for 45 min at 37 °C under continuous stirring (950 rpm) with 25 µL of a 2.0 µg mL⁻¹ AbS9.6 solution (prepared in PBS, pH 7.5).

After two washing steps with 50 µL of PBS, AbS9.6-coated MBs were re-suspended in 25 µL of the hybridization mixture solution and incubated during 45 min (950 rpm, 25 °C); after two washing steps with 50 µL of the commercial blocker casein solution the DNA/miRNA-AbS9.6-MBs were re-suspended in 25 µL of a 0.004 U mL⁻¹ Strep-HRP solution (prepared also in commercial blocker casein solution) and incubated for 30 min (37 °C, 950 rpm). The HRP-tagged DNA/miRNA-AbS9.6-MBs assembly was then washed two times with 50 µL of commercial blocker casein solution.

Finally, the modified-MBs were re-suspended in 45 µL of 0.05 M sodium phosphate buffer solution (pH 6.0) to perform the amperometric detection.

### 7. Amperometric measurements.

To perform the amperometric measurements the SPCE was positioned on a homemade casing of Teflon with neodymium magnet encapsulated, and the 45 µL of the modified MBs suspension were magnetically captured on the SPCE. Then, the magnet holding block was immersed into an electrochemical cell containing 10 mL 0.05 M phosphate buffer of pH 6.0 and 1.0 mM HQ (prepared just before performing the electrochemical measurement). Amperometric measurements in stirred solutions were made by applying a detection potential of -0.20 V vs. Ag pseudo-reference electrode upon addition of 50 µL of a 0.1 M H₂O₂ solution until the steady-state current was reached (approx. 100 s). The amperometric signals given through the manuscript corresponded to the difference between the steady-state and the background currents.

### 8. Simultaneous determination of miR-21 and miR-205 in one single experiment

Dual miRNAs determination was accomplished by simultaneously preparing two different batches of MBs each of them suitable for the determination of each target miR following the protocol described above for the determination of only one miRNA.

Thereafter, the modified MBs were re-suspended in 5 µL of pH 6.0 of 0.05 M sodium phosphate buffer solution (pH 6.0). The amperometric measurements at the dual SPCEs were performed as follows: the 5 µL of the resuspended MBs modified for miR-21 determination were magnetically captured on one of the working electrodes of the dual SPCEs (WE 1 in Figure 6) and the 5 µL suspension of the modified MBs for miR-205 determination were captured on the second working electrode (WE 2 in Figure 6) by keeping the dual SPCE in an horizontal position after placing it in the corresponding homemade magnet holding block.

### Example 2. Development of the MBs-based electrochemical biosensors of the invention and implementation of the detection protocol of miRNA-21 and miRNA-205 by using said sensor.

The electrochemical sensors based on MBs described in the present example, are based on the formation in solution of RNA/DNA heteroduplexes containing the RNA or DNA sequence of interest (originally single-stranded or previously denatured if double-stranded) and selective capture of such DNA/RNA hybrids by using superparamagnetic particles (MBs) labeled with an antibody or a fragment thereof capable of recognizing said RNA-DNA heteroduplexes.

To fabricate the MBs-based electrochemical sensors developed, in the first place, the hybridization between the target miRNA (variable concentration of miRNA-21 or miRNA-205) and the biotinylated antiDNA probe (0.05 µM of antiDNA-21 or antiDNA-205) was performed in homogeneous solution (in PBS, pH 7.5, by 45 min incubation at 37 °C and 950 rpm). Secondly the antibody AbS9.6 capable of recognizing RNA-DNA heteroduplexes was immobilized through its Fc region to ProtG-functionalized magnetic beads (by incubating 2.5 µL of ProtG-MBs with 25 µL of a 2.0 µg mL⁻¹ AbS9.6 solution prepared in PBS, pH 7.5, for 45 min at 37 °C and 950 rpm) and used as capture receptor for the DNA/miRNA duplex formed previously in solution (by incubating the AbS9.6-MBs with 25 µL of the hybridization mixture during 45 min at 37 °C and 950 rpm). Subsequently, the attached biotinylated hybrid was labeled with a commercial Strep-HRP polymer (by incubating the DNA/miRNA-AbS9.6-MBs with 25 µL of a 0.004 U mL⁻¹ Strep-HRP solution prepared in commercial blocker casein solution for 30 min at 37 °C and 950 rpm). To perform the electrochemical transduction, the modified MBs (re-suspended in 45 µL of 0.05 M sodium phosphate buffer solution, pH 6.0) were then magnetically captured on a solid support comprising a solid electrode made of carbon, and a neodymium magnet. After immobilizing the resulting MB in the electrodes, the detection of the hybridization process was performed by amperometry using the system hydroquinone (HQ) as redox mediator and H₂O₂, as enzymatic substrate. The added hydrogen peroxide was reduced in the presence of HRP, and the regeneration of the reduced form of the enzyme was mediated by HQ_{red} (Martin et al., 2014. J. Mater. Chem. B, 2: 739-746). The HQₒₓ generated is electrochemically reduced when the potential applied is more negative than the formal potential of the redox HQ_{red}/HQₒₓ pair.

Basically the procedure followed in the present example and illustrated in figure 1 involves four main steps: (i) homogeneous hybridization of the synthetic DNA probe and the target miRNA; (ii) capture of the resultant DNA/miRNA duplex by the AbS9.6-modified MBs; (iii) enzymatic labeling of the biotinylated DNA/miRNA duplex captured onto the AbS9.6-modified MBs and (iv) electrochemical detection of the modified-MBs on SPCEs. By using this procedure, up to 30 sensors a day can be worked (or more if the process is automated), illustrating the use of the present methodology for the rapid, simple, specific, quantitative and ultrasensitive detection and identification (detection limit [LOD] = 2.4 pM) of polynucleotides (endogenously single-stranded or previously denatured if naturally double-stranded) such as the miRNAs present in the given sample.

The detection limits obtained are illustrated in the examples below, showing the great sensitivity of the sensors of the present invention as devices with relevant usefulness in the analysis of complex samples, in particular in the analysis of miRNAs in clinical samples. In the present analysis and as shown thought-out the present document, the presence of other miRNAs in the analyzed sample did not interfere with the detection and identification of the miRNA illustrated herein.

### Example 3. Protocols for the detection of miRNA-205 using the biosensor of the invention.

### 3.1 Extended protocol

An electrochemical biosensor based on the efficient and selective hybridization in homogeneous solution of the target miRNA-205 to a fully complementary synthetic DNA probe (antiDNA-205) followed by the efficient and selective capture of the antiDNA-205/miRNA-205 duplex formed in solution using MBs modified with AbS9.6 was prepared as illustrated in example 1.

For the detection and quantification of miRNA-205, protocols described in details in Subsections 4 and 5 of Example 1 were followed.

In table I below we show the optimized experimental variables for the detection of miR-205 according to the extended protocol.

**Table I. Optimized experimental variables for the detection of miRNA-205.**

| | |
|---|---|
| ProtG-MBs, µL | 2.5 |
| [AbS9.6], µg mL⁻¹ | 2.0 |
| [bp-DNA], µM | 0.05 |
| Strep-HRP, dil | 1/25,000 |
| t_{incAbS9.6}, min | 45 |
| t_{hibridization bp-DNA/miRNA}, min | 45 |
| t_{AbS9.6-MBs+DNA/miRNA}, min | 45 |
| t_{Strep-HRP}, min | 30 |
| T^{a}_{incubation}, °C | 37 |

The protocol shown herein was performed in a total assay time of aprox. 75 min (once we prepared the Ab S9.6-MBs).

The operational and analytical characteristics of the methodology shown herein are summarized in table II below and in figure 2.

**Table II. Operational and analytical characteristics of the methodology shown in example 3.1.**

| | |
|---|---|
| r | 0.998 |
| Slope, nA ·nM⁻¹ | 9548 ± 211 |
| Intercept, nA | 173 ± 27 |
| Linear range, pM | 8.2-250 |
| Limit of detection (LOD), pM | 2.4 |
| Limit of quantification (LQ), pM | 8.2 |

As it is summarized in the table, the calibration graph constructed for the synthetic miRNA-205 target under the optimized experimental conditions (displayed in figure 2) showed linearity (r=0.998) between 8.2 and 250 pM, with a sensitivity of (9548 ± 211) nA nM⁻¹ and a limit of detection (LOD, estimated as 3×s_{b}/m, in which s_{b} is the standard deviation of the blank, and m is the slope of the calibration curve) of 2.4 pM (6 attomoles in 25 µL of sample), without the use of any amplification technique.

### 3.2. Reduced protocol

In order to improve the protocol as described in example 3.1 and reduce the assay time below 75 min, electrochemical biosensors were prepared as illustrated in said example. For the detection and quantification of miRNA-205, the protocol of 3.1 was modified only by shortening the times of the following incubation steps:
- Hybridization between the target miRNA-205 and the biotinylated antiDNA-205 from 45 to 15 min.
- Capture of the antiDNA-205/miRNA-205 duplex formed in solution by AbS9.6-MBs from 45 to 5 min.
- Label of the antiDNA-205/miRNA-205-AbS9.6-MBs with the Strep-HRP solution polymer from 30 to 5 min.

In table III below we show the total assay times of each of the steps of the reduced protocol

**Table III. Total assay times of the reduced protocol**

| | |
|---|---|
| t_{inc AbS9.6}, min | 45 |
| t_{hibridation bp-DNA/miRNA}, min | 15 |
| t_{AbS9.6-MBs + DNA/miRNA}, min | 5 |
| t_{Strep-HRP}, min | 5 |

The protocol shown herein was performed in a total assay time of approx. 30 min (once we prepared the AbS9.6-MBs). Figure 3 shows a comparison between the extended and reduced protocol, said comparison is also illustrated in table IV below

**Table IV. Comparison between the reduced and the extended protocol**

| | **Reduced protocol** | **Extended protocol** |
|---|---|---|
| r | 0.999 | 0.998 |
| Slope, nA ·nM⁻¹ | 5791 ± 131 | 9548 ± 211 |
| Intercept, nA | 89 ± 14 | 173 ± 27 |

The use of a reliable assay capable of providing results in approximately 30 minutes, highlights the usefulness of the present methodology in the analysis of clinical samples, in particular in the analysis of miRNAs in clinical samples.

### Example 4. Selectivity of the methodology developed.

The selectivity of the methodology developed, was evaluated by comparing the amperometric signal obtained with electrochemical biosensors fabricated using the extended protocol in the absence of target miRNA and in the presence of 0.5 nM of a fully complementary sequence (miRNA-205) of the DNA probe using in the assay (antiDNA-205), a single mismatched base (miRNA-205 1-m, sequence in Table 1) and 2 completely non-complementary sequences (miRNA-21 and miRNA-192, sequences in Table 1). The selectivity of the AbS9.6 to capture selectively only the DNA/RNA duplexes was also demonstrated by comparing the amperometric signals obtained in the absence and in the presence of 0.5 nM of the target miRNA-205 by performing the hybridization with the fully complementary antiDNA-205 or antimiRNA-205 probes.

**Table V. Selectivity of the developed methodology**

| **[miRNA], nM** | **-i, nA** |
|---|---|
| 0 | 137.8 |
| 0.5 miRNA-205 | 2522 |
| 0.5 miRNA-21 | 162.2 |
| 0.5 miRNA-205 1-m | 1283 |
| 0.5 miRNA-192 | 140.3 |

As it can be deduced from the results displayed in Figure 4a), the biosensor responses to the non-complementary sequences were not significantly different from that measured in the absence of target miRNA. Moreover, the 1-m sequence provided 51% of the response to the target miRNA-205. At this point, it is important to remark that the probability of finding in the same sample the complementary sequence and an equivalent with a single mismatched base is very low (Labib et al., J. Am. Chem. Soc. 135 (2013) 3027-3038.). Moreover, results presented in figure 4b) demonstrated the high affinity of the AbS9.6 only for DNA/RNA hybrids.

The results obtained herein clearly indicate the high selectivity of the methodology developed.

### Example 5. Reproducibility of the methodology developed.

The reproducibility of the methodology developed, was assayed by comparing the amperometric responses provided by 8 different biosensors fabricated in the same manner (extended protocol described in Section 3.1) for a 0.075 nM synthetic target miRNA-205 solution. The results, illustrated in the table below, gave a relative standard deviation (RSD) value of 3.1%, thus demonstrating a great reliability in the whole procedure involving both the biosensor fabrication protocol (MBs modification and magnetic capture on the SPCE surface) and the amperometric transduction.

**Table VI. Amperometric responses provided by each of the 8 different biosensors**

| **Electrode** | **-i, nA** |
|---|---|
| 1 | 943.4 |
| 2 | 923.9 |
| 3 | 905.6 |
| 4 | 913.2 |
| 5 | 979.7 |
| 6 | 977.3 |
| 7 | 912.7 |
| 8 | 920.5 |

### Example 6. Stability of the methodology developed.

The stability with time of the methodology developed was assayed by preparing the AbS9.6-MBs and storing them at 4 °C in Eppendorf tubes containing 50 µL of filtered PBS. Each working day one batch of the prepared conjugates was incubated a solution containing 0.075 nM of the synthetic target miRNA-205 according to the extended protocol. A control chart was constructed by setting the average current value calculated from 5 measurements made the first day of the study (day of the AbS9.6-MBs preparation) as the central value, while the upper and lower limits of control were set at ±3×SD of these initial values. The obtained results (shown in table VII) showed that the biosensor responses remained within the control limits for at least 29 days (no longer times were assayed). This good storage stability suggests the possibility of preparing sets of AbS9.6-MBs conjugates and storing them under the above mentioned conditions until their use for the biosensor preparation is required.

**Table VII. Amperometric responses provided by biosensors prepared using the stored AbS9.6-MBs.**

| **Days after preparation** | **-i, nA** |
|---|---|
| 0 | 834.9 |
| 4 | 874.9 |
| 16 | 993.9 |
| 18 | 879.3 |
| 22 | 865.1 |
| 25 | 913.8 |
| 29 | 882.7 |

### Example 7. Versatility of the methodology developed.

The present methodology is applicable to any miRNA, RNA or DNA single-stranded or double-stranded molecule of interest present in an isolated biological sample. To date we have assayed the present methodology for the determination of miRNA-205, miRNA-21, miR-223, miRNA-26b5p, miRNA-27a3p, miRNA-1273p and miRNA-933p.

The results obtained using the extended protocol and the DNA probe specific for each target miRNA (assayed at 0.25 nM concentration) are summarized in table VIII below.

**Table VIII. Amperometric responses provided by biosensors prepared using the extended protocol and the antiDNA probe specific for each target miRNA (assayed at 0.25 nM concentration)**

| **Target miR** | **-i, nA** |
|---|---|
| miRNA-205 | 1591 |
| miRNA-21 | 1071 |
| miRNA-223 | 1245 |
| miRNA-26b5p | 1040 |
| miRNA-27a3p | 1654 |
| miRNA-1273p | 1301 |
| miRNA-933p | 1372 |

### Example 8. Usefulness of the methodology developed in complex clinical samples.

The usefulness of the methodology developed was evaluated by detecting the endogenous level of a target miRNA in RNAₜ extracted from cell lines and human serum samples. These analyses were performed by using the extended protocol described in Section 3.1 but changing the synthetic target standard by the appropriate amount of RNAₜ extracted from the samples under study (prepared in PBS, pH 7.5).

The results are illustrated in tables IX-XI below

**Table IX. Determination of the endogenous content of miRNA-21 in RNAₜ extracted from cell lines.**

| **Cell line** | **RNAₜ, ng** | **-i, nA** |
|---|---|---|
| | 0 | 143.3 |
| | 50 | 189.9 |
| MCF-7 | 100 | 254.6 |
| | 250 | 533.7 |
| | 500 | 687.1 |
| | 1000 | 856.8 |
| MCF-10A | 500 | 243.6 |

As shown the methodology developed allows the detection of miRNA-21 using only 50 ng of RNAₜ.

**Table X. Determination of the endogenous content of miRNA-205 in RNAₜ extracted from cell lines.**

| **Cell line** | **RNAₜ, ng** | **-i, nA** |
|---|---|---|
| | 0 | 144.0 |
| MCF-7 | 50 | 156.8 |
| | 100 | 169.0 |
| | 500 | 145.6 |
| | 50 | 173.6 |
| MCF-10A | 100 | 200.5 |
| | 500 | 291.3 |

As demonstrated data summarized in Tables IX and X results provided by the magnetic beads-based biosensor showed over-expression of miRNA-21 and reduced expression of miRNA-205 in the metastatic breast cell line (MCF-7) compared to the normal-like MCF-10A cell line, which was in good agreement with that reported earlier (Ribas et al., Trends in Anal. Chem. 49 (2013) 20-30), (Campuzano et al., Biosens. Bioelectron. 66 (2015) 385-391), (Elgamal et al., PLos ONE 8(10) 2013: e76402. doi:10.1371/journal.pone.0076402).

Very interesting results were also obtained by applying the developed biosensor to the analysis of miRNA-21 in RNAₜ extracted from human serum samples, where miRNA-21 was highly expressed only in samples from breast cancer patients compared to those form healthy women.

**Table XI. Determination of the endogenous content of miR-21 in 500 ng of RNAₜ extracted from human serum samples.**

| **Woman** | **-i, nA** |
|---|---|
| Healthy 1 | 111.3 |
| With breast cancer | 299.1 |
| Healthy 2 | 114.6 |

### Example 9. Comparative of the different methodologies developed.

Different protocols (described in detail in table XII) were assayed to test the interaction between the AbS9.6 and the antiDNA/miRNA duplex. Figure 5 shows the amperometric responses measured for 0 and 0.25 nM of synthetic target miRNA-205 as well as the corresponding calculated S/N ratios. As it can be seen, although it is possible to detect the presence of the target miRNA using all the protocols, the largest S/N ratio was achieved when the AbS9.6 was used immobilized on the surface of MBs and when the labeling of the DNA/RNA duplex is performed when the duplex was previously captured by the AbS9.6-MBs. These results can be attributed to a poorer immobilization of the AbS9.6 onto the MBs after duplex recognition and to a hindered recognition of the DNA/RNA duplex previously labeled with the Strep-HRP polymer by the AbS9.6-MBs. Consequently, the 4-step protocol, involving selective recognition of the DNA/RNA duplex formed previously in solution by the premodified AbS9.6-MBs and the final enzymatic labeling of the duplex captured onto the AbS9.6-MBs, was selected in order to achieve the best sensitivity.

**Table XII. Protocols evaluated**

| **Protocol** | |
|---|---|
| **Optimized** | 1) Immobilization of AbS9.6 onto ProtG-MBs → AbS9.6-MBs |
| | 2) Homogenous hybridization of the target miRNA with the corresponding biotinylated antiDNA probe → b-DNA/RNA duplex |
| | 3) Capture of the b-DNA/RNA duplex by the AbS9.6-MBs |
| | 4) Labeling of the b-DNA/RNA-AbS9.6-MBs with Strep-HRP |
| **A** | 1) Homogenous hybridization of the target miRNA with the corresponding biotinylated antiDNA probe → b-DNA/RNA duplex |
| | 2) Capture of the b-DNA/RNA duplex with the AbS 9.6 in solution → b-DNA/RNA-AbS9.6 |
| | 3) Immobilization of the b-DNA/RNA-AbS9.6 onto ProtG-MBs |
| | 4) Labeling of the b-DNA/RNA-AbS9.6-MBs with Strep-HRP |
| **B** | 1) Homogenous hybridization of the target miRNA with the corresponding biotinylated antiDNA probe and capture of the b-DNA/RNA duplex with the AbS 9.6 in solution → b-DNA/RNA-AbS9.6 |
| | 2) Immobilization of the b-DNA/RNA-AbS9.6 onto ProtG-MBs |
| | 3) Labeling of the b-DNA/RNA-AbS9.6-MBs with Strep-HRP |
| **C** | 1) Immobilization of AbS9.6 onto ProtG-MBs → AbS9.6-MBs |
| | 2) Homogenous hybridization of the target miRNA with the corresponding biotinylated antiDNA probe → b-DNA/RNA duplex |
| | 3) Labeling of the b-DNA/RNA duplex with Strep-HRP in solution → HRP-DNA/RNA duplex |
| | 4) Capture of the HRP-DNA/RNA duplex by AbS9.6-MBs |

### Example 10. Simultaneous detection of two different miRNAs in one single experiment

Taking into account the interest in the multiplexed determination of miRNAs in clinical samples we evaluated also the potential of the methodology developed in this invention to simultaneously detect the expression of two different miRNAs in one single experiment at dual SPCEs.

The fundamentals of the simultaneous determination of miR-21 and miR-205 using the disposable dual electrochemical magnetic beads-based biosensor are displayed in Figure 6. In brief, two different batches of antiS9.6-MBs were prepared and used as capture receptor for the miRNA-21-antiDNA-21 or miRNA-205-antiDNA-205 heteroduplexes previously formed in solution by homogeneous hybridization of the specific and biotinylated antiDNA probe with the single-stranded (ss) target miR. Subsequently, the captured biotinylated hybrid was labeled with Strep-HRP polymer. The MBs bearing the miRNA-DNA/antiS9.6 complex for each target miR were magnetically captured on the corresponding working electrode (WE 1 and WE 2) of the dual SPCE and amperometric detection at - 200 mV of the catalytic current produced upon H₂O₂ addition using HQ as redox mediator in solution was employed to monitor the target miRNAs concentration.

## Claims

1. Method to detect and/or quantify miRNA, RNA or DNA molecules of interest in at least one isolated biological sample, through an electrochemical sensor based on the use of magnetic beads (magnetic beads-based electrochemical sensor), comprising the following sequential steps:
a. Obtaining a RNA/DNA, DNA/DNA or RNA/RNA hybrid by carrying out a homogenous hybridization in solution of a miRNA, RNA or DNA molecule of interest present in the isolated biological sample to a complementary RNA or DNA sequence labeled with a compound A, to provide the RNA/DNA, DNA/DNA or RNA/RNA hybrid;
b. Capturing the product of the hybridization in solution of step a) by adding to said solution a suspension of magnetic particles, preferably superparamagnetic particles (MBs), previously bound to an antibody or a fragment thereof capable of recognizing RNA-DNA heteroduplexes or DNA-DNA or RNA-RNA duplexes through a protein G coupled to the surface of the magnetic particles, wherein said product of the hybridization is captured onto the said magnetic particles;
c. Adding an element capable of being detected and/or quantified by a magnetic beads-based electrochemical sensor, wherein said element binds to compound A, thereby generating a complex capable of being detected and/or quantified by a magnetic beads-based electrochemical sensor;
d. Magnetic capturing the product of step c) comprising the magnetic particles onto an electrochemical support; and
e. detecting and/or quantifying a electrochemical signal produced by the complex of step d), thereby detecting and/or quantifying the product of hybridization of step a) and thereby detecting and/or quantifying the miRNA, RNA or DNA molecules of interest present in the at least one biological sample;
wherein the antibody fragment is selected from the group consisting of "Fab" fragments, "F(ab')2" fragments, "Fv" fragments, single chain Fv fragments or "scFv", "Diabodies" and "bispecific antibodies" (Bab);
wherein "compound A" is any compound capable of giving an electrochemical signal after binding with another compound;
wherein "electrochemical support" is any rigid or flexible support which can function as a working electrode in an electrochemical transduction; and
wherein an element capable of being detected and/or quantified by a magnetic beads-based electrochemical biosensor is an element capable of producing an electrochemical reaction by itself or after reaction with other molecules.

2. The method of claim 1, wherein the molecule of interest is a miRNA molecule.

3. The method of claim 2, wherein compound A is a compound capable of binding with a compound selected from the group consisting of biotin, streptavidin, avidin, digoxigenine and fluorescein.

4. The method according to any of the precedent claims, wherein the antibody or fragment thereof is selected from the list consisting of antipoly(A)-poly-(dt) and S9.6.

5. The method of any of the precedent claims, wherein the element capable of being detected and/or quantified by a magnetic beads-based electrochemical biosensor is a complex formed by an element capable of binding to compound A and an enzyme capable of being detected and/or quantified by a magnetic beads-based electrochemical sensor.

6. The method of claim 5 wherein said enzyme is an oxidase or a hydrolase such as phosphatase alkaline, preferably a peroxidase, more preferably the horseradish peroxidase (HRP) enzyme, and said detection and/or quantification is carried out by monitoring the enzymatic reduction of an enzymatic substrate system (substrate and co-substrate), preferably H₂O₂ and hydroquinone.

7. The method of any of the precedent claims, wherein the isolated biological sample is a tissue sample or a biological fluid such as blood, serum, plasma, cerebrospinal fluid, saliva or urine.

8. The method of any of the precedent claims, wherein the electrochemical sensor of step d) comprises i) a solid electrode made of a material selected from the list consisting of: gold, carbon, platinum, CD-trode, screen-printed electrodes, silver, mercury, graphite, glassy carbon, carbon nanotubes, gold nanowires, gold nanoparticles, metallic oxide nanoparticles, carbon paste, boron-doped diamond and composites, and ii) a magnet, preferably a neodymium magnet.

9. *In vitro* use of an antibody or a fragment thereof, wherein said antibody or fragment thereof is bound to magnetic particles, preferably superparamagnetic particles (MBs), through a protein G coupled to the surface of the magnetic particles, and wherein said antibody or fragment thereof is capable of recognizing RNA-DNA heteroduplexes or DNA-DNA or RNA-RNA duplexes, for implementing the method as defined in any of claims 1 to 8.

10. *In vitro* use of a composition, preferably in the form of a suspension, comprising the antibody or fragment thereof as defined in claim 9, for implementing the method as defined in any of claims 1 to 8.

11. *In vitro* use of a kit or device for detecting and/or quantifying miRNAs, RNA or DNA molecules of interest in at least one isolated biological sample by using a magnetic beads-based electrochemical sensor, which comprises:
a. the antibody or fragment thereof bound to magnetic particles of claim 9;
b. a RNA or DNA molecule labeled with a compound A complementary to the miRNAs, RNA or DNA molecules of interest;
c. an element capable of being detected and/or quantified by a magnetic beads-based electrochemical biosensor, wherein said element binds to compound A; and
d. optionally a solution of the enzymatic substrate system (substrate and co-substrate), preferably H₂O₂ and hydroquinone;
for implementing the method as defined in any of claims 1 to 8;
wherein "compound A" is any compound capable of giving an electrochemical signal after binding with the element of step c); and
wherein an element capable of being detected and/or quantified by a magnetic beads-based electrochemical biosensor is an element capable of producing an electrochemical reaction by itself or after reaction with other molecules.

12. The use of the kit or device of claim 11, wherein said kit or device further comprises a solid support which in turn comprises i) a solid electrode made of a material selected from the list consisting of: gold, carbon, platinum, CD-trode, screen-printed electrodes, silver, mercury, graphite, glassy carbon, carbon nanotubes, gold nanowires, gold nanoparticles, metallic oxide nanoparticles, carbon paste, boron-doped diamond and composites, and ii) a magnet, preferably a neodymium magnet.

13. The use of the kit or device of claim 12, wherein the solid electrode of i) is made of carbon, wherein the antibody or fragment thereof is capable of recognizing RNA-DNA heteroduplexes and is selected from the list consisting of antipoly(A)-poly-(dt) and S9.6 and wherein the molecule of interest is a miRNA.

## Patentansprüche

1. Verfahren zum Nachweisen und/oder Quantifizieren von miRNS-, RNS- oder DNS-Molekülen von Interesse in mindestens einer isolierten biologischen Probe, durch einen elektrochemischen Sensor basierend auf der Verwendung von magnetischen Perlen (*magnetic beads-based electrochemical sensor*), umfassend die folgenden sequenziellen Schritte:
a. das Erhalten eines RNS/DNS, DNS/DNS oder RNS/RNS-Hybrides mittels des Durchführens eines homogenen Lösungshybridisierung eines miRNS-, RNS- oder DNS-Moleküls von Interesse vorhanden in der isolierten biologischen Probe mit einer komplementären RNS- oder DNS-Sequenz markiert mit einer Verbindung A, um das RNS/DNS-, DNS/DNS- oder RNS/RNS-Hybrid bereitzustellen;
b. das Abfangen des Produkts der Lösungshybridisierung aus Schritt a) durch das Hinzufügen zur genannten Lösung einer Aufschlämmung von magnetischen Partikeln, vorzugsweise superparamagnetischen Partikeln (MB), welche zuvor mit einem Antikörper oder einem Fragment desselben gebunden wurden, welcher in der Lage ist, RNS-DNS-Heteroduplexen oder DNS-DNS- oder RNS-RNS-Duplexen durch ein mit der Oberfläche der magnetischen Partikel gekoppeltes G-Protein zu erkennen, wobei das genannte Produkt der Hybridisierung auf den genannten magnetischen Partikeln abgefangen wird;
c. das Hinzufügen eines Elements, welches in der Lage ist, von einem elektrochemischen Sensor basierend auf magnetischen Perlen nachgewiesen und/oder quantifiziert zu werden, wobei sich das genannte Element mit Verbindung A bindet, wodurch ein Komplex erzeugt wird, welcher in der Lage ist, von einem elektrochemischen Sensor basierend auf magnetischen Perlen nachgewiesen und/oder quantifiziert zu werden;
d. das magnetische Abfangen des Produkts aus Schritt c) umfassend die magnetischen Partikeln auf einem elektrochemischen Träger; und
e. das Nachweisen und/oder Quantifizieren eines elektrochemischen Signals hergestellt durch den Komplex aus Schritt d), wodurch das Produkt der Hybridisierung aus Schritt a) nachgewiesen und/oder quantifiziert wird und dadurch die miRNS-, RNS- oder DNS-Molekülen von Interesse nachgewiesen und/oder quantifiziert werden, welche in der mindestens einen biologischen Probe vorhanden sind;
wobei das Antikörperfragment aus der Gruppe bestehend aus "Fab"-Fragmenten, "F(ab')2"-Fragmenten, "Fv"-Fragmenten, einzelkettigen Fv-Fragmenten oder "scFv", "Diakörpern" und "bispezifischen Antikörpern" (Bab) ausgewählt wird;
wobei "Verbindung A" jeder Verbindung ist, welche in der Lage ist, ein elektrochemisches Signal nach der Bindung mit einer anderen Verbindung auszugeben;
wobei der "elektrochemische Träger" jeder starre oder flexible Träger ist, welcher als Arbeitselektrode in einer elektrochemischen Transduktion wirken kann; und
wobei ein Element, welches in der Lage ist, von einem elektrochemischen Biosensor basierend auf magnetischen Perlen nachgewiesen und/oder quantifiziert zu werden, ein Element ist, welches in der Lage ist, selber eine elektrochemischen Reaktion oder nach der Reaktion mit anderen Molekülen zu erzeugen.

2. Verfahren nach Anspruch 1, wobei das Molekül von Interesse ein miRNS-Molekül ist.

3. Verfahren nach Anspruch 2, wobei Verbindung A eine Verbindung ist, welcher in der Lage ist, sich mit einer Verbindung ausgewählt aus der Gruppe bestehend aus Biotin, Streptavidin, Avidin, Digoxigenin und Fluoreszein zu binden.

4. Verfahren nach einem der vorhergehenden Ansprüche, wobei der Antikörper oder Fragment desselben aus der Liste bestehend aus Antipoly(A)-poly-(dt) und S9.6 ausgewählt wird.

5. Verfahren nach einem der vorhergehenden Ansprüche, wobei das Element, welches in der Lage ist, von einem elektrochemischen Biosensor basierend auf magnetischen Perlen nachgewiesen und/oder quantifiziert zu werden, ein Komplex ist, welcher von einem Element gebildet wird, welches in der Lage ist, sich mit Verbindung A zu binden und ein Enzym, welches in der Lage ist, von einem elektrochemischen Sensor basierend auf magnetischen Perlen nachgewiesen und/oder quantifiziert zu werden

6. Verfahren nach Anspruch 5, wobei das genannte Enzym eine Oxydase oder eine Hydrolase ist, wie alkalische Phosphatase, vorzugsweise eine Peroxydase, weiter vorzugsweise das Meerrettichperoxydase (HRP)-Enzym, und wobei das genannte Nachweisen und/oder die genannte Quantifizierung durch das Überwachen der enzymatischen Reduktion eines enzymatischen Substratsystem (Substrat und Cosubstrat), vorzugsweise H₂O₂ und Hydrochinon durchgeführt wird.

7. Verfahren nach einem der vorhergehenden Ansprüche, wobei die isolierte biologische Probe eine Gewebeprobe oder ein biologisches Fluid ist, wie Blut, Serum, Plasma, Gehirn-Rückenmark-Flüssigkeit, Speichel oder Urin.

8. Verfahren nach einem der vorhergehenden Ansprüche, wobei der elektrochemische Sensor aus Schritt d) i) eine feste Elektrode hergestellt aus einem Material ausgewählt aus der Liste bestehend aus: Gold, Kohlenstoff, Platin, CD-Elektrode, Siebdruckelektroden, Silber, Quecksilber, Graphit, Glaskohlenstoff, Kohlenstoff-Nanorohren, Gold-Nanodrähten, Gold-Nanopartikeln, Metalloxid-Nanopartikeln, Kohlenstoffpaste, bordotiertem Diamant und Verbundmaterialien, und ii) einen Magnet, vorzugsweise einen Neodym-Magnet, umfasst.

9. *In Vitro*-Verwendung eines Antikörpers oder eines Fragments desselben, wobei der genannte Antikörper oder Fragment desselben mit den magnetischen Partikeln, vorzugsweise superparamagnetischen Partikeln (MB), durch ein mit der Oberfläche der magnetischen Partikeln gekoppeltes G-Protein gebunden sind, und wobei der genannte Antikörper oder Fragment desselben in der Lage ist, RNS-DNS-Heteroduplexen oder DNS-DNS- oder RNS-RNS-Duplexen zu erkennen, zum Verwirklichen des Verfahrens nach einem der Ansprüche 1 bis 8.

10. *In Vitro*-Verwendung einer Zusammensetzung, vorzugsweise in Form einer Aufschlämmung, umfassend den Antikörper oder Fragment desselben nach Anspruch 9, zum Verwirklichen des Verfahrens nach einem der Ansprüche 1 bis 8.

11. *In Vitro*-Verwendung eines Kits oder einer Vorrichtung zum Nachweisen und/oder Quantifizieren von miRNS-, RNS- oder DNS-Molekülen von Interesse in mindestens einer isolierten biologischen Probe unter Verwendung eines elektrochemischen Sensors basierend auf magnetischen Perlen, welche Folgendes umfasst:
a. den Antikörper oder Fragment desselben, welcher/welches mit magnetischen Partikeln nach Anspruch 9 gebunden ist;
b. ein RNS- oder DNS-Molekül markiert mit einer mit den miRNS-, RNS- oder DNS-Molekülen von Interesse komplementären Verbindung A;
c. ein Element, welches in der Lage ist, von einem elektrochemischen Biosensor basierend auf magnetischen Perlen nachgewiesen und/oder quantifiziert zu werden, wobei das genannte Element sich mit Verbindung A bindet; und
d. optional eine Lösung des enzymatischen Substratsystem (Substrat und Cosubstrat), vorzugsweise H₂O₂ und Hydrochinon;
zum Verwirklichen des Verfahrens nach einem der Ansprüche 1 bis 8;
wobei die "Verbindung A" jede Verbindung ist, welche in der Lage ist, ein elektrochemisches Signal nach der Bindung mit dem Element aus Schritt c) auszugeben; und
wobei ein Element, welches in der Lage ist, von einem elektrochemischen Biosensor basierend auf magnetischen Perlen nachgewiesen und/oder quantifiziert zu werden, ein Element ist, welches in der Lage ist selber eine elektrochemische Reaktion oder nach der Reaktion mit anderen Molekülen zu erzeugen.

12. Verwendung des Kits oder der Vorrichtung nach Anspruch 11, wobei das genannte Kit oder die genannte Vorrichtung zusätzlich einen festen Träger umfasst, welcher wiederum i) eine feste Elektrode hergestellt aus einem Material ausgewählt aus der Liste bestehend aus: Gold, Kohlenstoff, Platin, CD-Elektrode, Siebdruckelektroden, Silber, Quecksilber, Graphit, Glaskohlenstoff, Kohlenstoff-Nanorohren, Gold-Nanodrähten, Gold-Nanopartikeln, Metalloxid-Nanopartikeln, Kohlenstoffpaste, bordotiertem Diamant und Verbundmaterialien, und ii) einen Magnet, vorzugsweise einen Neodym-Magnet, umfasst.

13. Verwendung des Kits oder der Vorrichtung nach Anspruch 12, wobei die feste Elektrode aus i) aus Kohlenstoff hergestellt ist, wobei der Antikörper oder Fragment desselben in der Lage ist, RNS-DNS-Heteroduplexen zu erkennen und aus der Liste bestehend aus Antipoly(A)-poly-(dt) und S9.6 ausgewählt wird und wobei das Molekül von Interesse eine miRNS ist.

## Revendications

1. Procédé de détection et/ou quantification de molécules d'intérêt de miRNA, RNA ou DNA dans au moins un échantillon biologique isolé, à travers un capteur électrochimique basé sur l'utilisation de billes magnétiques (capteur électrochimique basé sur des billes magnétiques), comprenant les étapes séquentielles suivantes:
a) l'obtention d'un hybride RNA/DNA, DNA/DNA ou RNA/RNA en mettant en oeuvre une hybridation homogène en solution d'une molécule d'intérêt de miRNA, RNA ou DNA présente dans l'échantillon biologique isolé dans une séquence complémentaire RNA ou DNA avec un composé A, pour obtenir l'hybride RNA/DNA, DNA/DNA ou RNA/RNA;
b) la capture du produit de l'hybridation en solution de l'étape a) par l'addition à ladite solution d'une suspension de particules magnétiques, de préférence des particules supeparamagnétiques (MBs), préalablement reliées à un anticorps ou un fragment de celles-ci capable de reconnaître des hétéroduplexes RNA-DNA ou des duplexes DNA-DNA ou RNA-RNA à travers une protéine G couplée à la surface des particules magnétiques, dans lequel ledit produit de l'hybridation est capturé sur lesdites particules magnétiques;
c) l'addition d'un élément apte à être détecté et/ou quantifié par un capteur électrochimique basé sur des billes magnétiques, dans lequel ledit élément est relié, au composant A, en générant ainsi un complexe apte à être détecté et/ou quantifié par un capteur électrochimique basé sur des billes magnétiques;
d) la capture magnétique du produit de l'étape c) comprenant les particules magnétiques sur un support électrochimique; et
e) la détection et/ou quantification d'un signal électrochimique produit par le complexe de l'étape d), en détectant et/ou quantifiant ainsi le produit d'hybridation de l'étape a) et en détectant et/ou quantifiant ainsi les molécules d'intérêt de miRNA, RNA ou DNA présentes dans au moins un échantillon biologique;
dans lequel le fragment d'anticorps est choisi parmi le groupe composé de fragments «Fab», fragments «F(ab')2», fragments «Fv», des fragments Fv de chaîne simple ou «scFv», des «diabodies» et des «anticorps bispécifiques» (Bab);
dans lequel «composé A» est tout composé apte à fournir un signal électrochimique après son union avec un autre composé;
dans lequel «support électrochimique» est tout support rigide ou flexible qui peut fonctionner comme une électrode de travail dans une transduction électrochimique; et
dans lequel un élément apte à être détecté et/ou quantifié par un biocapteur électrochimique basé sur des billes magnétiques est un élément apte à produire une réaction électrochimique par lui-même ou après une réaction avec d'autres molécules.

2. Procédé selon la revendication 1, dans lequel la molécule d'intérêt est une molécule miRNA.

3. Procédé selon la revendication 2, dans lequel le composé A est une composé apte à se relier à un composé choisi parmi le groupe composé de biotine, streptavidine, avidine, digoxigénine et fluorescéine.

4. Procédé selon l'une quelconque des revendications précédentes, dans lequel l'anticorps ou le fragment de celui-ci est choisi parmi la liste composée d'antipoly(A)-poly-(dt) et S9.6.

5. Procédé selon l'une quelconque des revendications précédentes, dans lequel l'élément apte à être détecté et/ou quantifié par un biocapteur électrochimique basé sur des billes magnétiques est un complexe formé par un élément apte à se relier à un composé A et une enzyme apte à être détectée et/ou quantifiée par un capteur électrochimique basé sur des billes magnétiques.

6. Procédé selon la revendication 5 dans lequel ladite enzyme est une oxydase ou une hydrolase telle que phosphatase alcaline, de préférence une peroxydase, avantageusement l'enzyme de la peroxydase du raifort (HRP), et ladite détection et/ou quantification est mise en oeuvre en contrôlant la réduction enzymatique d'un système de substrats enzymatiques (substrat et co-substrat), de préférence H₂O₂ et hydroquinone.

7. Procédé selon l'une quelconque des revendications précédentes, dans lequel l'échantillon biologique isolé est un échantillon tissulaire ou un fluide biologique tel que du sang, sérum, plasma, fluide céphalo-rachidien, salive ou urine.

8. Procédé selon l'une quelconque des revendications précédentes, dans lequel le capteur électrochimique de l'étape d) comprend i) une électrode solide faite en une matière choisie parmi la liste composée de: or, carbone, platine, CD-trode, électrodes imprimées par sérigraphie, argent, mercure, graphite, carbone vitreux, nanotubes de carbone, nanofils d'or, nanoparticules d'or, nanoparticules d'oxyde métallique, pâte de carbone, diamant dopé au bore et composites et ii) un aimant, de préférence un aiment de néodyme.

9. Utilisation *in vitro* d'un anticorps ou un fragment de celui-ci, dans lequel ledit anticorps ou fragment de celui-ci est relié à des particules magnétiques, de préférence des particules superparamagnétiques (MBs), à travers une protéine G couplée à la surface des particules magnétiques, et dans laquelle ledit anticorps ou fragment de celui-ci est apte à reconnaître des hétéroduplexes RNA-DNA ou des duplexes DNA-DNA ou RNA-RNA, pour mettre en oeuvre le procédé défini selon l'une quelconque des revendications 1 - 8.

10. Utilisation *in vitro* d'une composition, de préférence en forme d'une suspension, comprenant l'anticorps ou fragment de celui-ci défini dans la revendication 9, pour mettre en oeuvre le procédé défini dans l'une quelconque des revendications 1 à 8.

11. Utilisation *in vitro* d'une trousse ou dispositif de détection et/ou quantification de molécules d'intérêt de miRNA, RNA ou DNA dans au moins un échantillon biologique isolé en utilisant un capteur électrochimique basé sur des billes magnétiques, qui comprend:
a. l'anticorps ou fragment de celui-ci relié aux particules magnétiques de la revendication 9;
b. une molécule RNA ou DNA marquée par un composé A complémentaire aux molécules d'intérêt miRNA, RNA ou DNA;
c. un élément apte à être détecté et/ou quantifié par un biocapteur électrochimique basé sur des billes magnétiques, dans laquelle ledit élément est relié au composé A; et
d. optionnellement une solution du système de substrats enzymatiques (substrat et co-substrat), de préférence H₂O₂ et hydroquinone;
pour mettre en oeuvre le procédé défini dans l'une quelconque des revendications 1 - 8;
dans lequel «composé A» est tout composé apte à fournir un signal électrochimique après son union avec l'élément de l'étape c); et;
dans lequel un élément apte à être détecté et/ou quantifié par un biocapteur électrochimique basé sur des billes magnétiques est un élément apte à produire une réaction électrochimique par lui-même ou après une réaction avec d'autres molécules.

12. Utilisation de la trousse ou dispositif selon la revendication 11, dans laquelle ladite trousse ou dispositif comprend en outre un support solide qui comprend à son tour i) une électrode solide faite d'une matière choisie parmi la liste composée de: or, carbone, platine, CD-trode, électrodes imprimées par sérigraphie, argent, mercure, graphite, carbone vitreux, nanotubes de carbone, nanofils d'or, nanoparticules d'or, nanoparticules d'oxyde métallique, pâte de carbone, diamant dopé au bore et composites et ii) un aimant, de préférence un aiment de néodyme.

13. Utilisation de la trousse ou dispositif selon la revendication 12, dans laquelle l'électrode solide de i) est faite en carbone, dans laquelle l'anticorps ou fragment de celui-ci est apte à reconnaître des hétéroduplexes RNA-DNA et est choisie parmi la liste composée d'antipoly(A)-poly-(dt) et S9.6 et dans laquelle la molécule d'intérêt est un miRNA.


### SEQUENCE LISTING

<110> CANAAN Research & Investment S.L.
<120> Magnetic beads-based electrochemical biosensor
<130> 901 904
<160> 17
<170> PatentIn version 3.5
<210> 1
   <211> 21
   <212> RNA
   <213> Artificial Sequence
<220>
   <223> AntimiRNA-205 Probe
<220>
   <221> misc_feature
   <222> (1)..(21)
<400> 1
   agacuccggu ggaaugaagg a 21
<210> 2
   <211> 21
   <212> DNA
   <213> Artificial Sequence
<220>
   <223> antiDNA-205 Probe
<220>
   <221> misc_feature
   <222> (1)..(21)
<400> 2
   agactccggt ggaatgaagg a 21
<210> 3
   <211> 21
   <212> RNA
   <213> Artificial Sequence
<220>
   <223> Target miRNA-205
<220>
   <221> misc_feature
   <222> (1)..(21)
<400> 3
   uccuucauuc caccggaguc u 21
<210> 4
   <211> 22
   <212> DNA
   <213> Artificial Sequence
<220>
   <223> antiDNA-21 Probe
<220>
   <221> misc_feature
   <222> (1)..(22)
<400> 4
   tcaacatcag tctgataagc ta 22
<210> 5
   <211> 19
   <212> RNA
   <213> Artificial Sequence
<220>
   <223> Target miRNA-21
<220>
   <221> misc_feature
   <222> (1)..(19)
<400> 5
   uagcuuauca gacugaugu 19
<210> 6
   <211> 22
   <212> DNA
   <213> Artificial Sequence
<220>
   <223> antiDNA-223 Probe
<220>
   <221> misc_feature
   <222> (1)..(22)
<400> 6
   aactcagctt gtcaaataca cg 22
<210> 7
   <211> 22
   <212> RNA
   <213> Artificial Sequence
<220>
   <223> Target miRNA-223
<220>
   <221> misc_feature
   <222> (1)..(22)
<400> 7
   cguguauuug acaagcugag uu 22
<210> 8
   <211> 22
   <212> DNA
   <213> Artificial Sequence
<220>
   <223> AntiDNA-26b5p
<220>
   <221> misc_feature
   <222> (1)..(22)
<400> 8
   tacctatcct gaattacttg aa 22
<210> 9
   <211> 22
   <212> RNA
   <213> Artificial Sequence
<220>
   <223> Target miRNA-26b5p
<220>
   <221> misc_feature
   <222> (1)..(22)
<400> 9
   uucaaguaau ucaggauagg ua 22
<210> 10
   <211> 22
   <212> DNA
   <213> Artificial Sequence
<220>
   <223> AntiDNA-27a3p
<220>
   <221> misc_feature
   <222> (1)..(22)
<400> 10
   tgcggaactt agccactgtg aa 22
<210> 11
   <211> 22
   <212> RNA
   <213> Artificial Sequence
<220>
   <223> Target miRNA-27a3p
<220>
   <221> misc_feature
   <222> (1)..(22)
<400> 11
   uucacagugg cuaaguuccg ca 22
<210> 12
   <211> 22
   <212> DNA
   <213> Artificial Sequence
<220>
   <223> AntiDNA-1273p
<220>
   <221> misc_feature
   <222> (1)..(22)
<400> 12
   agccaagctc agacggatcc ga 22
<210> 13
   <211> 22
   <212> RNA
   <213> Artificial Sequence
<220>
   <223> Target miRNA-1273p
<220>
   <221> misc_feature
   <222> (1)..(22)
<400> 13
   ucggauccgu cugagcuugg cu 22
<210> 14
   <211> 22
   <212> DNA
   <213> Artificial Sequence
<220>
   <223> AntiDNA-933p
<220>
   <221> misc_feature
   <222> (1)..(22)
<400> 14
   cgggaagtgc tagctcagca gt 22
<210> 15
   <211> 22
   <212> RNA
   <213> Artificial Sequence
<220>
   <223> Target miRNA-933p
<220>
   <221> misc_feature
   <222> (1)..(22)
<400> 15
   acugcugagc uagcacuucc cg 22
<210> 16
   <211> 21
   <212> RNA
   <213> Artificial Sequence
<220>
   <223> 1-mismatched miRNA-205 (miRNA-205 1-m)
<220>
   <221> misc_feature
   <222> (1)..(21)
<400> 16
   uccuucauua caccggaguc u 21
<210> 17
   <211> 21
   <212> RNA
   <213> Artificial Sequence
<220>
   <223> miRNA-192
<220>
   <221> misc_feature
   <222> (1)..(21)
<400> 17
   cugaccuaug aauugacagc c 21